# EUROPEAN PATENT SPECIFICATION

(11) **EP 4 405 021 B1**
(45) Date of publication and mention of the grant of the patent: **13.08.2025**
(21) Application number: 22789438.3
(22) Date of filing: 22.09.2022
(51) Int. Cl.: A61M 25/00, A61M 25/01, A61B 1/005, A61B 17/00

(54) **ROTATIONAL AND DEFLECTABLE CATHETER CONTROL ASSEMBLY**
STEUEREINHEIT EINES DREH- UND LENKBAREN KATHETERS
ENSEMBLE DE COMMANDE DE CATHÉTER ROTATIF ET DÉVIABLE

(30) Priority: 22.09.2021 US 202163246835 P
(43) Date of publication of application: 31.07.2024
(73) Proprietor: Entellect Medical Holdings, Louisville, Kentucky 40222 (US)
(72) Inventor: APPLING, Anthony, Crestwood, Kentucky 40014 (US); MORRIS, Ben, Jeffersonville, Indiana 47130 (US); WOOLEY, Chase, New Albany, Indiana 47150 (US)
(74) Representative: Becker, Eberhard
(86) International application number: PCT/US2022/076833
(87) International publication number: WO 2023/049783

(56) References cited:
- US-A1- 2013 184 528
- US-A1- 2021 235 973
- US-B2- 11 116 942

## Description

### TECHNICAL FIELD

The present disclosure relates to a control assembly for a medical interventional device, such as catheter. More particularly, the present disclosure relates to a control assembly capable of providing both rotation and deflection of a distal tip of a catheter in response to movement of at least one actuator dial by a user.

### BACKGROUND OF THE INVENTION

A catheter is a medical instrument for use in accessing an interior of a patient's body with a distal tip during a medical procedure. The catheter can include at least one working component, such as a fluid channel, a working channel, and/or an electronics cable, which extend along the catheter and terminate at or adjacent the distal tip. Catheters typically have a control handle which is configured to allow a user to control a position of the distal tip during the procedure, such as via rotation of a knob on the control handle to effectuate rotation of the catheter and the distal tip about an axis. However, the working components which pass through the catheter shaft are prone to winding, overlapping, occlusion or other damage during their simultaneous rotation with the catheter shaft. Torsional stresses and crossing of the working components caused during rotation of the catheter shaft can damage or impair the function of the catheter device. For example, the fluid channel is utilized to deliver fluids and the working channel is utilized to deliver an instrument to the distal tip of the catheter during the medical procedure. However, kinking, crossing or other occlusion of these channel components can prevent the related fluids or instrument from reaching the distal tip during the medical procedure, and thus resulting in an ineffective catheter that fails during the medical procedure. Additionally, knotting or worse yet facture of the electronics cable can lead to full loss of function of the electronics. Thus, there remains a need for improvements to such catheter control assemblies in which the catheter shaft is rotatable about the axis during the medical procedure by a user and includes at least one working component.

An example of a control assembly for a catheter having a channel with a static and a dynamic portion is known from US2021235973A1.

### SUMMARY OF THE INVENTION

A control assembly for a catheter having at least one working component includes a housing extending along an axis between a proximal housing end and a distal housing end to define a housing compartment extending therebetween. A control case is disposed within the housing compartment adjacent the proximal housing end. A catheter shaft extends within the housing compartment from the control case to a distal tip disposed adjacent the distal housing end. The catheter shaft is rotatable about the axis during operation of the control assembly to effectuate rotation of the distal tip. At least one working component of the catheter includes at least one channel component extending from a static portion disposed adjacent the proximal housing end in coupled and generally stationary relationship relative to the control case to a dynamic portion extending along and rotatable about the axis simultaneously with the catheter shaft. A lumen interruption mechanism is disposed in the control case and extends from a proximal mechanism end disposed in communication with the static portion of the at least one channel component to a distal mechanism end disposed in communication with the dynamic portion of said at least one channel component. As will be more fully explained in the following detailed description, the lumen interruption mechanism transitions the at least one channel component from the static portion to the dynamic portion as the at least one channel component translates through the housing compartment and is dynamically rotated about the axis simultaneously with the catheter shaft to maintain the integrity of the dynamic portion of the at least one channel component, and avoid damage such as via kinking, occlusion, or the like during use of the control assembly in a medical procedure.

### BRIEF DESCRIPTION OF THE DRAWINGS

Other aspects of the present disclosure will be readily appreciated, as the same becomes better understood by reference to the following detailed description when considered in connection with the accompanying drawings wherein:
Figure 1 is a perspective view of a control assembly including a housing extending from a proximal housing end to a distal housing end along an axis A and a handle portion disposed adjacent the proximal housing end and axially translatable along the housing towards the distal housing end;
Figure 1A is a cross-sectional view of the control assembly illustrating a control case disposed within the housing adjacent the proximal housing end and a catheter shaft extending from a distal case end of the control case to a distal tip disposed adjacent the distal housing end;
Figure 2 is a perspective view of the control case illustrating a lumen interruption mechanism disposed adjacent a proximal case end, a center gear disposed adjacent the distal case end, a synchronous rotation mechanism operably coupling a union reservoir hub of the lumen interruption mechanism and the center gear for establishing simultaneous and synchronous rotation of these components, and a cable take-up assembly;
Figure 3 is a perspective view of the lumen interruption mechanism illustrating the union reservoir hub rotatably coupled to a static, union reservoir cap and including the cable take-up assembly;
Figure 4 is an exploded perspective view of the lumen interruption mechanism;
Figure 5 is a top view of the lumen interruption mechanism;
Figure 6 is a bottom view of the lumen interruption mechanism;
Figure 7 is a cross-sectional view of the lumen interruption mechanism illustrating a sealed fluid communication path extending through the union reservoir cap and hub sequentially via a cap fluid inlet, a fluid reservoir and a cap fluid outlet;
Figure 8 is a cross-sectional view of the lumen interruption mechanism illustrating a fluid tight working channel extending axially through the union reservoir cap and hub between a central cap orifice and a central hub orifice;
Figure 9 is a partial cross-sectional perspective view of the control case illustrating the union reservoir hub disposed adjacent the proximal case end and with the union reservoir cap removed to more clearly illustrate the circumferentially shaped fluid reservoir;
Figure 10 is a perspective view of the union reservoir hub and the cable-take-up assembly;
Figure 11A is a cross-sectional view of the lumen interruption mechanism illustrating operation of the cable take-up assembly during rotation about the axis in a first rotational direction;
Figure 11B is a cross-sectional view of the lumen interruption mechanism illustrating operation of the cable take-up assembly during rotation about the axis in a second rotational direction opposite the first rotational direction;
Figure 12 is a perspective view of a lead screw;
Figure 13 is a side view of an anchor arm and an anchor shaft;
Figure 14 is a cross-sectional view of the anchor arm and the anchor shaft;
Figures 15A, B and C illustrate various cross-sectional views of the control assembly as the control case and the handle portion disposed in surrounding relationship with the control case translate axially towards the distal housing to illustrate a proportional support mechanism consistently supporting the catheter shaft at a midpoint between the center gear and the distal end of the housing as the catheter shaft advances with the control case along the axis and the distal end of the catheter shaft exits the distal housing end;
Figure 16 is a perspective view of the proportional support mechanism illustrating a catheter support arm extending from a proximal support arm end operably coupled to a reduction gear to a distal support arm end, and a catheter support platform extending radially inwardly from the distal support arm end to define a catheter lumen for supporting the catheter shaft;
Figure 17 is a transparent perspective view of a portion of the housing illustrating a minor gear rack of the catheter support arm operably coupled to a minor gear feature of the reduction gear and a major gear rack extending along the housing operably coupled to a major gear feature of the reduction gear;
Figure 18A is an exploded perspective view of the proportional support mechanism as viewed from a first side of Figures 16 and 17 to more clearly illustrate a gear boss extending from the distal case end and the minor gear rack extending between the catheter support arm between the proximal and distal support arm ends;
Figure 18B is an exploded perspective view of the proportional support mechanism as viewed from a second opposite side of Figures 16 and 17 to more clearly illustrate the minor gear feature of the reduction gear for mating with the minor gear rack, and the major gear rack extending along the housing;
Figure 19 is a cross-sectional view of the control case illustrating alternative embodiments of the synchronous rotation mechanism and the cable take-up assembly;
Figure 20 is a magnified perspective view of a portion of Figure 19 more clearly illustrating the alternative embodiment of the cable take-up assembly;
Figure 21 is a perspective view of a loop of the alternative embodiment of the cable take-up assembly;
Figures 22A, B and C illustrate cross-sectional views of the control case sequentially demonstrating operation of the second embodiment of the cable take-up assembly from a static condition (Figure 22A) to rotation about the axis in a first rotational direction (Figure 22B) and then rotation about the axis in a second rotational direction opposite the first rotational direction (Figure 22C);
Figure 23 is a cross-sectional view of the control case of Figure 19 additionally illustrating the union reservoir cap and hub of the lumen interruption mechanism, the threaded gear, the lead screw, the anchor arm and the center gear in cross-section to more clearly illustrate the alternative embodiment of the synchronous rotation mechanism including an axle extending from a proximal axle end directly connected to the union reservoir hub and a distal axle end directly connected to the center gear for establishing simultaneous and synchronous rotation of these directly connected components;
Figure 24 is a magnified perspective cross-sectional view of a portion of Figure 19;
Figure 25 is a perspective view of the axle in the alternative embodiment of the synchronous rotation mechanism; and
Figures 26A-B are perspective cross-sectional views of the control case illustrating a lead screw and an anchor arm axially displaced in the proximal direction relative to the center gear (as shown in Figure 26B) in response to rotation of a threaded gear operably coupled to a deflection control knob to apply a resultant tension to a pull wire secured to the anchor arm for deflecting the distal tip of the catheter shaft.

### DETAILED DESCRIPTION OF THE EXEMPLARY EMBODIMENTS

In the following description, details are set forth to provide an understanding of the present disclosure. In some instances, certain systems, structures and techniques have not been described or shown in detail in order not to obscure the disclosure.

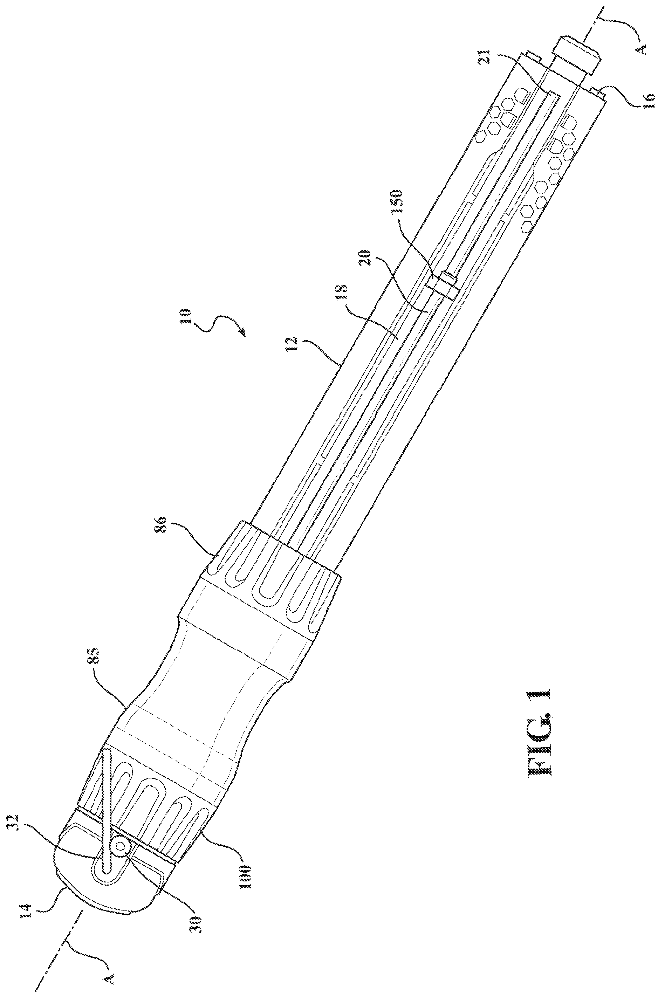

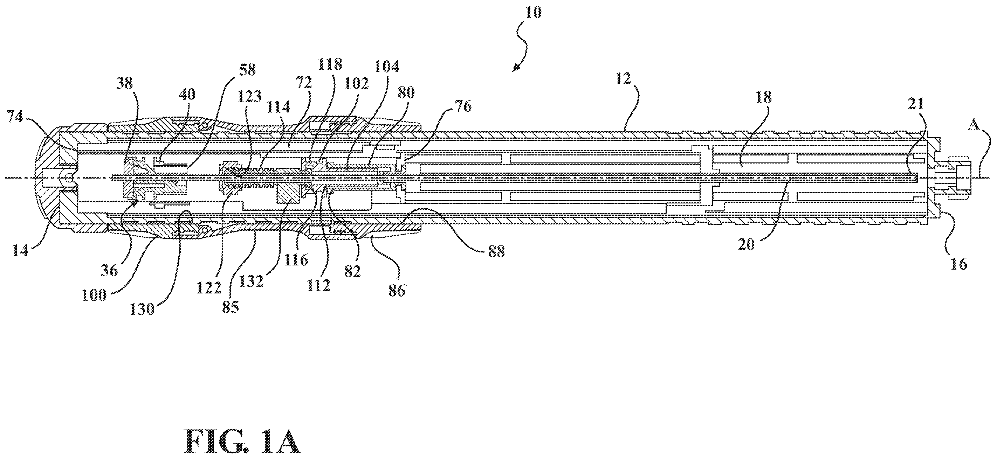

Referring to the Figures, wherein like numerals indicate corresponding parts throughout the several views, a control assembly **10** for a medical interventional device, such as a catheter, is generally shown. While the subject control assembly **10** is described herein for use with a catheter, it should be appreciated that the control assembly **10** could be used in association with other medical interventional devices without departing from the scope of the subject disclosure. As best illustrated in Figures 1 and 1A, the catheter control assembly **10** includes a housing **12** which extends in a longitudinal direction along an axis **A** between a proximal housing end **14** and a distal housing end **16** to define a housing compartment **18** for receiving the catheter, along with at least one of its associated working components, namely a working channel component **22,** a fluid channel component **24,** and/or an electronics cable **26.**

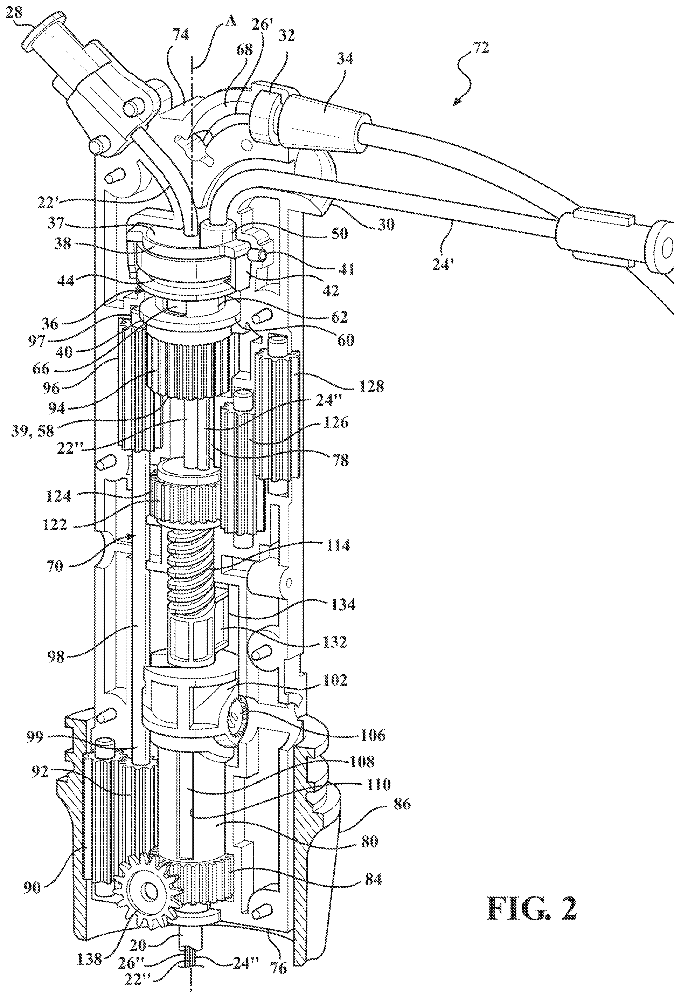

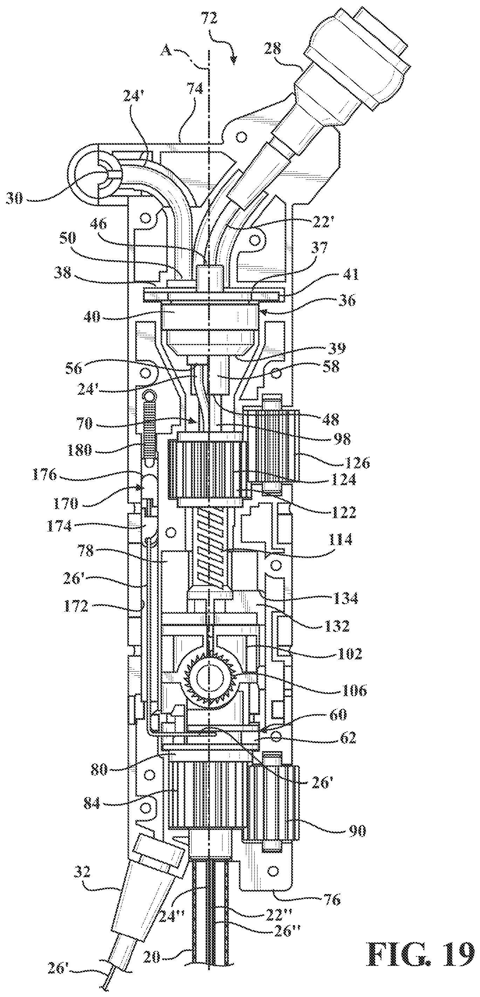

More specifically, and as further illustrated in Figures 1A, a control case **72** is disposed within the housing compartment **18** adjacent the proximal housing end **14,** and a catheter shaft **20** extends within the housing compartment **18** from the control case **72** and extending through and beyond the housing compartment to a distal tip **21** disposed adjacent and exterior to the distal housing end **16.** The working channel component **22** and the fluid channel component **24** of the catheter extend from respective static portions **22', 24'** disposed adjacent the proximal housing end **14** in coupled and generally stationary relationship relative to the control case **72** and the housing **12** during operation of the control assembly **10.** For example, as best illustrated in Figures 2 and 19, a proximal case end **74** of the control case **72** includes a working channel port **28** and a fluid channel port **30,** each for receiving respective static portions **22', 24'** of the working channel and fluid channel components **22, 24** from an environment of the control assembly **10.** The electronics cable component **26** also extends from a static portion **26'** disposed in coupled and generally stationary relationship relative to the control case **72** and the housing **12** during operation of the control assembly **10.** For example, as also illustrated in Figure 2, the proximal case end **74** of the control case **72** can additionally include an electronics port **32** including a strain relief sheath **34** for receiving the static portion **26'** of the electronics cable **26** from the environment of the control assembly **10.** However, as illustrated in Figure 19, the electronics port **32** can also be disposed adjacent the distal case end **76** of the control case **72** such that the static portion **26'** of the electronics cable **26** enters the control case **72** and is coupled generally stationary relative to the distal case end **76** without departing from the scope of the subject disclosure.

Each of the static portions **22', 24', 26'** of the working channel component **22,** the fluid channel component **24** and the electronics cable **26** communicate with respective dynamic portions **22", 24", 26"** that extend along and are rotatable about the axis A simultaneously with the catheter shaft **20.** In other words, the catheter shaft **20** houses a dynamic portion **22"** of the working channel component **22,** a dynamic portion **24"** of the fluid channel component **24,** and a dynamic portion **26"** of the electronics cable **26** that pass through the catheter shaft **20** and break out at one or more locations adjacent or at the distal tip **21** of the catheter shaft **20.**

As noted previously, for a catheter shaft **20** that rotates during operation of the control assembly 10 in response to control by a user, it is problematic for the dynamic portions **22", 24", 26"** of the working channel, fluid channel and electronics cable components **22, 24, 26** (i.e., those portions of the working channel **22,** fluid channel **24** and electronics cable **26** passing through the compartment **18** of the housing **12** and along the catheter shaft **20)** to wind or overlap during rotation of the catheter shaft **20.** Torsional stresses and the crossing of the dynamic portions of these working components **22, 24, 26** during rotation can lead to damage or impair the function of the catheter. Accordingly, the control assembly **10** includes a lumen interruption mechanism **36** disposed adjacent the proximal housing end **14** of the control assembly **10** for transitioning the working components **22, 24, 26** from their static portions **22', 24', 26'** as they enter the control assembly **10** to their dynamic portions **22", 24", 26"** as they translate through the compartment **18** of the housing **12** and are dynamically rotated about the axis **A** simultaneously with the catheter shaft **20** to achieve rotational control of the distal tip **18** during a procedure.

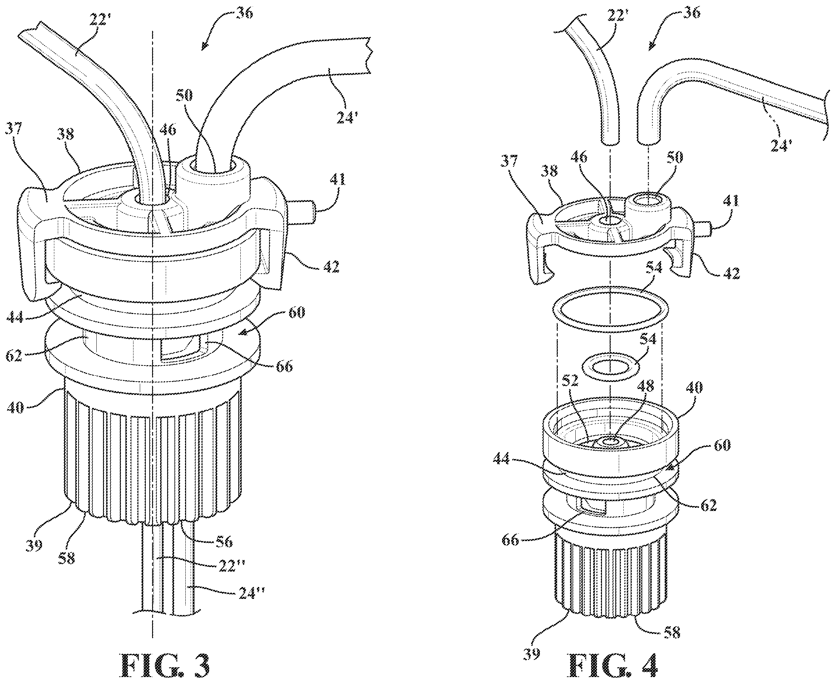

As best illustrated in Figures 3-4, 7-8 and 19, the lumen interruption mechanism **36** is disposed in the control case **72** adjacent the proximal case end **74** and extends from a proximal mechanism end **37** disposed in communication with the static portions **22', 24'** of the working channel component **22** and the fluid channel component **24** to a distal mechanism end **39** disposed in communication with the dynamic portions **22", 24"** of the working channel component **22** and the fluid channel component **24** for transitioning the working channel and fluid channel components **22, 24** from their static to dynamic portions. The lumen interruption mechanism **36** includes a union reservoir cap **38** that is static (i.e., disposed in a generally fixed/non-movable condition) relative to the housing **12** of the control assembly **10** and a union reservoir hub **40** that is rotatably coupled to the union reservoir cap **38** and dynamically free to fully rotate about the axis **A** and relative to the union reservoir cap **38** within the control handle **10** and the control case **72.** As best illustrated in Figure 3 and 19, the union reservoir cap **38** includes a post **41** that mates with a corresponding portion of the control handle **38** to keep the union reservoir cap **38** from rotating about the axis A and maintain the union reservoir cap **38** in its static position. As also illustrated in Figures 3 and 4, in a preferred arrangement, the union reservoir cap **38** includes a pair of legs **42** that extend along an outer portion of the union reservoir hub **30** and terminate in a rotational channel **44** defined by the union reservoir hub **40** for rotatably coupling these two components together. However, other means of fixing the union reservoir cap **36** and establishing the rotatable coupling of the union reservoir cap and hub **36, 38** could be utilized without departing from the scope of the subject disclosure.

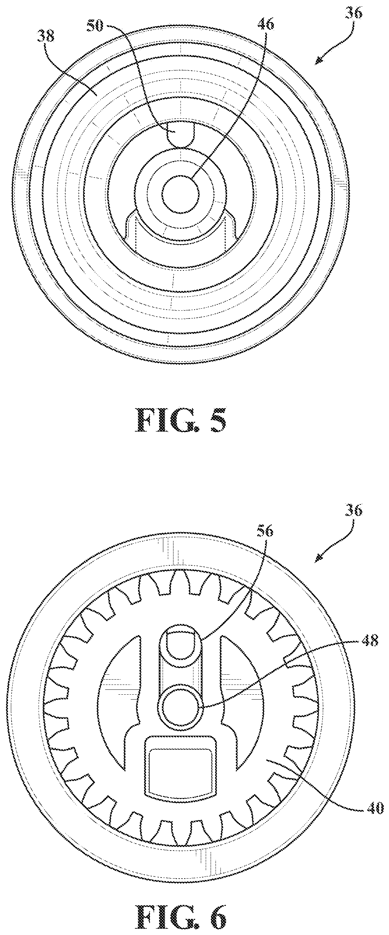

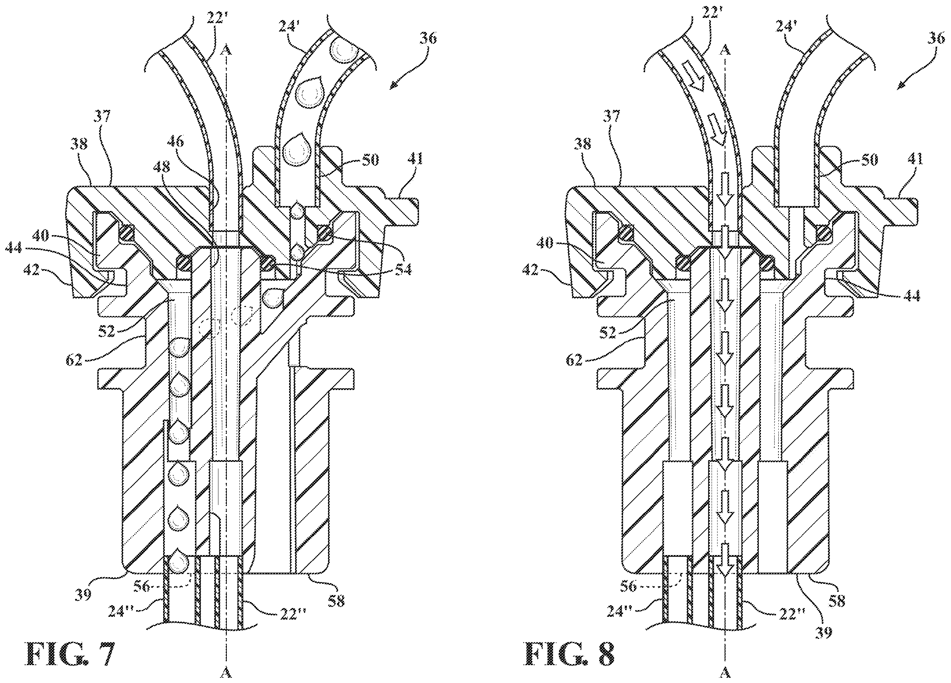

As best illustrated in Figures 4-8 and 19, the union reservoir cap **38** defines a central cap inlet **46** and the union reservoir hub **40** defines a central hub outlet **48.** Each of the central cap inlet **46** and central hub outlet **46, 48** extend through their respective components in aligned relationship with the axis **A** to dispose the inlet and outlet **46, 48** in aligned, fluid communication with one another when the union reservoir cap and hub **38, 40** are rotatably coupled together. With reference to Figure 8, the aligned central cap and hub inlet and outlet **46, 48** define a fluid tight working channel, with a uniform ID that extends through the lumen interruption mechanism **36.** The union reservoir cap **38** also defines a cap fluid inlet **50** disposed in radially offset relationship with the axis **A,** extending in generally parallel relationship with the central cap inlet **46.** The union reservoir hub **40** defines a fluid reservoir **52** extending circumferentially about the central hub outlet **48** and disposed adjacent the central reservoir cap **38** in fluid communication with the cap fluid inlet **50.** As best illustrated in Figures 4 and 7-8, at least one sealing device **54,** such as an o-ring, gasket, gland or the like, is disposed between the union reservoir cap and hub **38, 40** to seal the fluid reservoir **52.** A cap fluid outlet **56** extends in radially spaced and generally parallel relationship with the axis **A** from the fluid reservoir **52** to a bottom end **58** of the union reservoir hub **40.** Thus, as best illustrated in Figure 7, a fluid communication path is established through the rotatably coupled union reservoir cap and hub **38, 40** sequentially via the cap fluid inlet **50,** the fluid reservoir **52,** and the cap fluid outlet **54,** with fluid communication maintained between the cap fluid inlet and outlets **50, 54** even during rotation of the union reservoir hub **40** relative to the union reservoir cap **38** via the circumferntially-shaped fluid reservoir **52.**

As best illustrated in Figures 3, 7-8 and 19, the dynamic portion **22"** of the working channel component **22** extending from at or adjacent the distal end **21** of the catheter shaft **20** terminates at the bottom end **58** of the union reservoir hub **40** and is coupled to the central hub outlet **48.** Similarly, the dynamic portion **24"** of the fluid channel component **24** extending from at or adjacent the distal tip **21** of the catheter shaft **20** terminates at the bottom end **58** of the union reservoir hub **40** and is coupled to the hub fluid outlet **56.** As further illustrated in Figures 2-3 and 19, the static portion **22'** of the working channel component **22** extends from the working channel port **28,** terminates at the union reservoir cap **38** and is coupled with the central cap inlet **46.** Similarly, the static portion **24'** of the fluid channel component **24** extends from the fluid channel port **30,** terminates at the union reservoir cap **38** and is coupled to the cap fluid inlet **50.**

In operation, and as will be explained in more detail below, rotation of the catheter shaft **20** to effectuate rotational movement of the distal tip **21,** such as via rotation of a rotation control knob **86** on the control assembly **10** by a user, simultaneously and synchronously drives rotation of the union reservoir hub **40** relative to the union reservoir cap **38.** As a result, the dynamic portions **22", 24"** of the working and fluid channel components **22, 24** which each extend from the union reservoir hub **40** to the distal tip **21** of the catheter shaft **20** rotate simultaneously and synchronously with one another and the catheter shaft **20,** while the static portions **22', 24'** of the working and fluid channel components **22, 24** which extend from the union reservoir cap **38** to their respective ports **28, 30** remain in a static condition (notwithstanding rotational movement of the catheter shaft **20).** Thus, as illustrated in Figure 8, the lumen interruption mechanism **36** advantageously provides for a continuous ID working channel **22** that is static on the proximal mechanism end **37** of the lumen interruption mechanism **36** and torsionally dynamic on the distal mechanism end **39** of the lumen interruption mechanism **36,** while remaining fluid tight. The collective working channel, resulting from the combination of the static portion of the working channel **22',** the aligned central cup and hub inlet and outlet **46, 48,** as well as the dynamic portion of the working channel **22",** also remains centrally aligned on the axis **A** even during rotation of the catheter shaft **20.** Furthermore, as illustrated in Figure 7, the lumen interruption mechanism **36** allows for the passage of fluid therethrough from the cap fluid inlet **50** to the cap fluid outlet **56** through a secondary off-axis fluid communication path that is independent of the central, aligned and collective working channel extending along the axis **A.** In this way, static inputs to the lumen interruption mechanism **36** are not adversely affected by rotational movement of the catheter shaft **20** and kinking/occlusion/damage of the dynamic portions **22", 24"** of the working channel components extending from the lumen interruption mechanism **36** to the distal tip **21** of the catheter shaft **20** is avoided. The dynamic portion of the working channel **22"** and the dynamic portion of the fluid channel **24"** remain in their same relative positions to one another even while the catheter shaft **20** is being rotated about the axis **A** to effectuate rotation of the distal tip **21.** Thus, these working components **22, 24** do not wind or overlap when the catheter shaft **20** is rotating, improving the functionality of the control assembly **10** relative to prior art devices.

As previously mentioned, rotation of the catheter shaft **20** to effectuate rotational movement of the distal tip **21** simultaneously and synchronously drives rotation of the union reservoir hub **40** relative to the union reservoir cap **38.** Synchronicity of these dynamic rotating mechanisms prevents winding and kinking of the various dynamic portions of the working components emanating from the union reservoir hub **40.** By all elements rotating as "one single body" relative to the remaining components that make up the control assembly **10,** winding damage within the control assembly **10** is negated. As will be described in more detail below with reference to Figures 1A, 2 and 19, this relationship is accomplished through the use of a synchronous rotation mechanism **70.**

As best illustrated in Figures 1A, 2 and 19, the control case **72** extends from the proximal case end **74** to a distal case end **76** to define a control compartment **78** extending therebetween. The lumen interruption mechanism **36** is preferably disposed in the control compartment **78** adjacent the proximal case end **74.** The control case **72** includes a center gear **80** disposed in the control compartment **78** adjacent the distal case end **76** in rotatably aligned relationship with the central axis **A.** As best illustrated in Figures 1A, 2 and 19, the catheter shaft **20** is secured or coupled to the center gear **80** for rotation therewith (as will be described in more detail below), and the center gear **80** defines a center gear passageway **82** extending along the axis **A** for allowing the dynamic portions of the working channel **22,** fluid channel **24** and electronic cable **26** which extend from the catheter shaft **20** to pass through the center gear passageway **82,** along the control compartment **78** and into their coupled positions with the union reservoir hub **40.**

The center gear **80** defines a set of center gear teeth **84** extending radially outwardly from the center gear **80** in circumferentially aligned relationship with the central axis **A.** A handle portion **85** surrounds the control case **72** and includes the rotation control knob **86** rotatably disposed about the distal case end **76** of the control case **72.** The rotation control knob **86** includes a set of rotation knob gear teeth **88** circumferentially arranged along an inner diameter of the rotation control knob **86.** The rotation control knob **86** is oriented on the control case **72** such that its axis of rotation remains constant relative to the control assembly **10** and its axial position also remains constant. At least one spur gear **90, 92** is disposed between the rotation control knob **86** and the center gear **80** for disposing the rotation control knob **86** in operably coupled relationship with the set of center gear teeth **84** for driving rotation of the center gear **80** and the catheter shaft **20** in response to rotation of the rotation control knob **86** by a user.

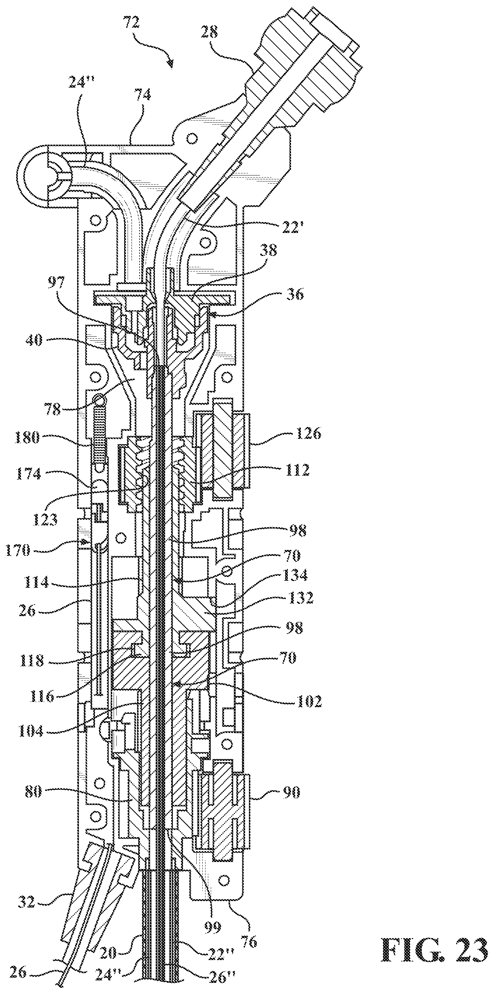

For example, as best illustrated in Figures 19 and 23, the control assembly **10** can include a single, inner spur gear **90** for establishing the operable coupled relationship between the rotation control knob **86** and the center gear **80.** However, as best illustrated in Figure 2, the control assembly could include the inner spur gear **90** plus an additional, adjacent spur gear **92** which are sequentially disposed between the rotation control knob **86** and the center gear **80** to establish the operable interconnection therebetween. The use of the additional, adjacent spur gear **92** allows rotation of the rotation control knob **86** to distribute rotation of the center gear **80** in the same rotational direction.

More specifically, as illustrated in Figure 2, each of the inner spur gear **90** and the adjacent spur gear **92** are disposed radially inward from the rotation control knob **86** such that their respective axis of rotation are aligned with one another, as well as the central axis **A,** in parallel (but radially spaced) relationship. As a result, the rotation knob gear teeth **88** are disposed in operable connection with the inner spur gear **90,** which is disposed in operable connection with the adjacent spur gear **92,** which is in operable connection with the center gear **80** to drive rotation of the catheter shaft **20** about the axis **A** in the same direction as the rotational direction of the rotation control knob **86.** The diameters of this power train are designed to a desired output ratio.

The synchronous rotation mechanism **70** is disposed in operably coupled relationship with the union reservoir hub **40** of the lumen interruption mechanism **36** and the center gear **80** to simultaneously and synchronously drive rotation of the union reservoir hub **40** in response to rotation of the center gear **80** (and the catheter shaft **20** coupled thereto) via the rotation control knob **86.** As best illustrated in Figures 2, 19 and 23, the synchronous rotation mechanism **70** includes an axle **98** extending from a proximal axle end **97** operably coupled with the union reservoir hub **40** to a distal axle end **99** operably coupled with the center gear **80.** The axle **98** is rotatable in response to rotation of the center gear **80** to establish the synchronous rotation of the union reservoir hub **40.**

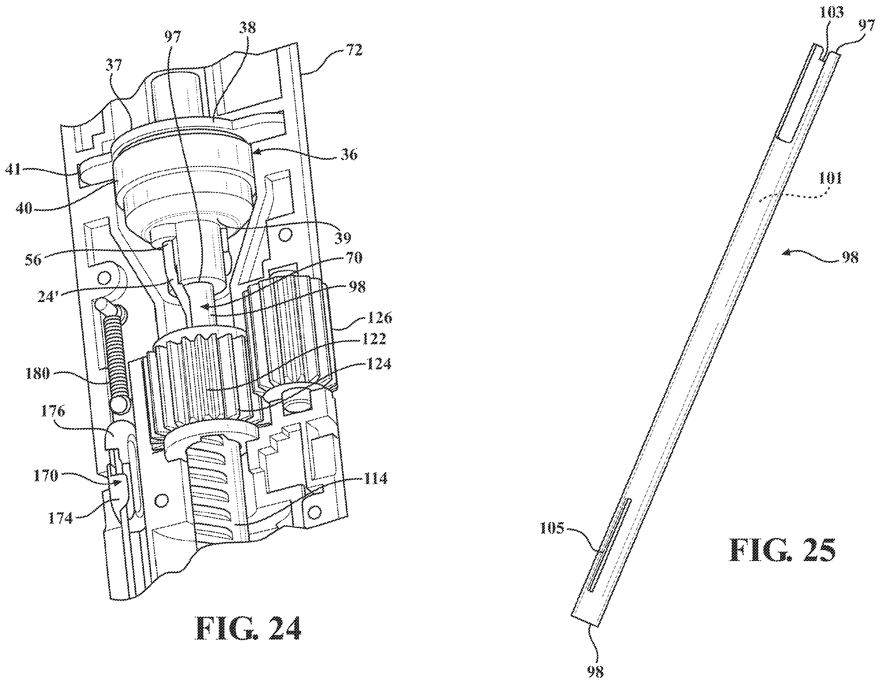

More specifically, as best illustrated in Figures 19, 23 and 25, in accordance with a first embodiment of the synchronous rotation mechanism **70,** the axle **98** extends within the control compartment **78** in aligned relationship with the axis **A** such that the proximal axle end **97** is directly connected to the union reservoir hub **40** and the distal axle end **99** is directly connected to the center gear **80.** In this arrangement, rotation of the center gear **80** by the rotation control knob **86** results in a direct drive of the union reservoir hub **40** via the axle **98.** The direct drive axle **98** is preferably comprised of a rigid tubular structure strong enough to distribute torque from the center gear **80** to which it is attached distally to the union reservoir hub **40** to which it is attached proximally. As will be appreciated in view of the second embodiment of the synchronous rotation mechanism **70,** use of the direct drive axle **98** simplifies a structure of the synchronous rotation mechanism **70** through use of a single component as opposed to requiring multiple components to establish synchronous rotation of the union reservoir hub **40** with the center gear **80.** As further illustrated in Figures 19-20 and 23, the arrangement of the tubular-shaped axle **98** along the axis A also allows the working channel **22,** the fluid channel **24,** and/or the electronics cable **26** to pass through the axle **98.** More specifically, the axle **98** defines an internal component passageway **101** extending between the proximal and distal axle end **97, 99,** such that the dynamic portions **22", 24"** of the working and fluid channel components **22, 24** can extend from their respective hub outlets **48, 54,** pass through the internal component passageway **101** and into the catheter shaft **20** (which is coupled to the center gear **80).** As will be described in more detail below, in an arrangement, the dynamic portion **26'** of the electronics cable **26** could also pass from the lumen interruption mechanism **36** and into the internal component passageway **101** for routing to the catheter shaft **20.** As further illustrated in Figure 25, the proximal axle end **97** of the axle **98** in the first embodiment of the synchronous rotation mechanism **70** can include a castellation feature **103** for establishing a mechanical interface and torque distribution to the union reservoir hub **40** via the direct connection. The distal axle end **99** of the axle can also include a breakout slot **105** for allowing any of the working components to break out of the interior component passageway **101** as needed to make their connections to the complementary components of the control assembly **10.**

As best illustrated in Figure 2, in accordance with a second embodiment of the synchronous rotation mechanism **70,** the proximal and distal axle ends **97, 99** of the axle **99** are indirectly connected to the union reservoir hub **38** and center gear **80,** respectively. More specifically, in this second embodiment of the synchronous rotation mechanism **70,** the union reservoir hub **40** defines a set of hub gear teeth **94** extending radially outwardly from the union reservoir hub **40** in circumferentially aligned relationship about the central axis **A.** The synchronous rotation mechanism **70** includes a proximal spur gear **96** disposed on the proximal axle end **97** of the axle **98,** radially outward from the union reservoir hub **40** and in operable engagement with the hub gear teeth **94.** The distal axle end **99** of the axle **98** is connected to the adjacent spur gear **92** that is operably interconnected with the center gear **80,** which is used to drive rotation of the center gear **80** via rotation of the rotation control knob **86** in accordance with an arrangement, as previously described. The proximal spur gear **96** is rotatable by the axle **98** about an axis of rotation that is parallel and radially spaced with the central axis **A,** and axially aligned with the axis of rotation of the adjacent spur gear **92.** Put another way, the proximal spur gear **96** and the adjacent spur gear **92** are disposed in axially aligned relationship for rotation about a shared axis of rotation. The axle **98** extends axially between the adjacent spur gear **92** and the proximal spur gear **96** along this shared axis of rotation to synchronously and simultaneously drive rotation of the proximal spur gear **96** via rotation of the adjacent spur gear **92** driven by the rotation control knob **86.** The proximal spur gear **96** is configured to have the same design and radial orientation as the adjacent spur gear **92** to result in simultaneous equivalent rotation. The hub gear teeth **94** of the union reservoir hub **40** are also identical in size, number and diameter to the center gear teeth of the center gear **80.** In this way, rotation of the center gear **80** results in 1: 1 rotation of the union reservoir hub **40** and therefore the catheter shaft **20** connected to the center gear **80** and the working components coupled to the union reservoir hub **40** move in synchronicity in response to rotation of the rotation control knob **86** by a user of the control assembly **10.**

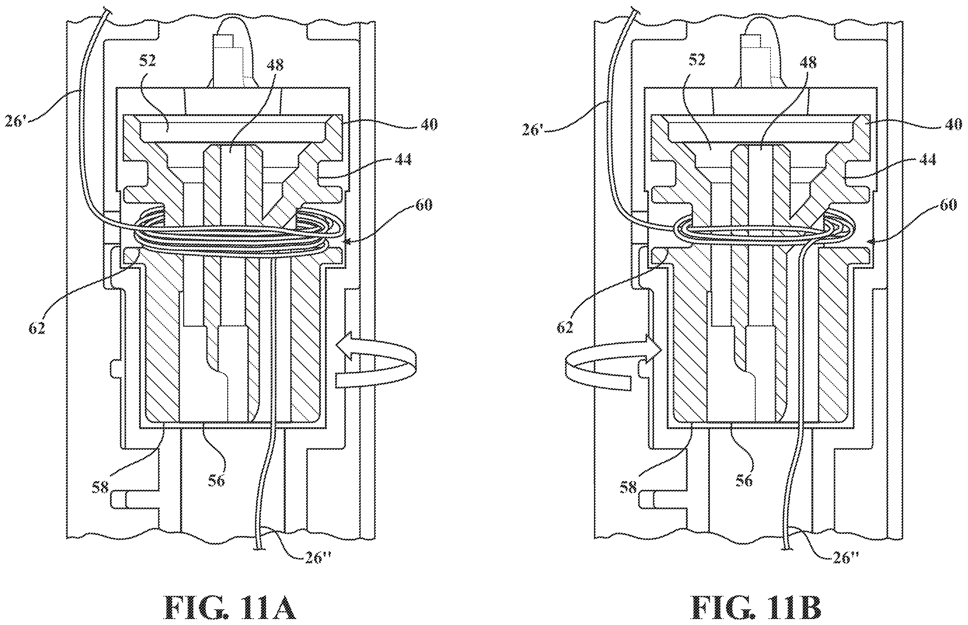

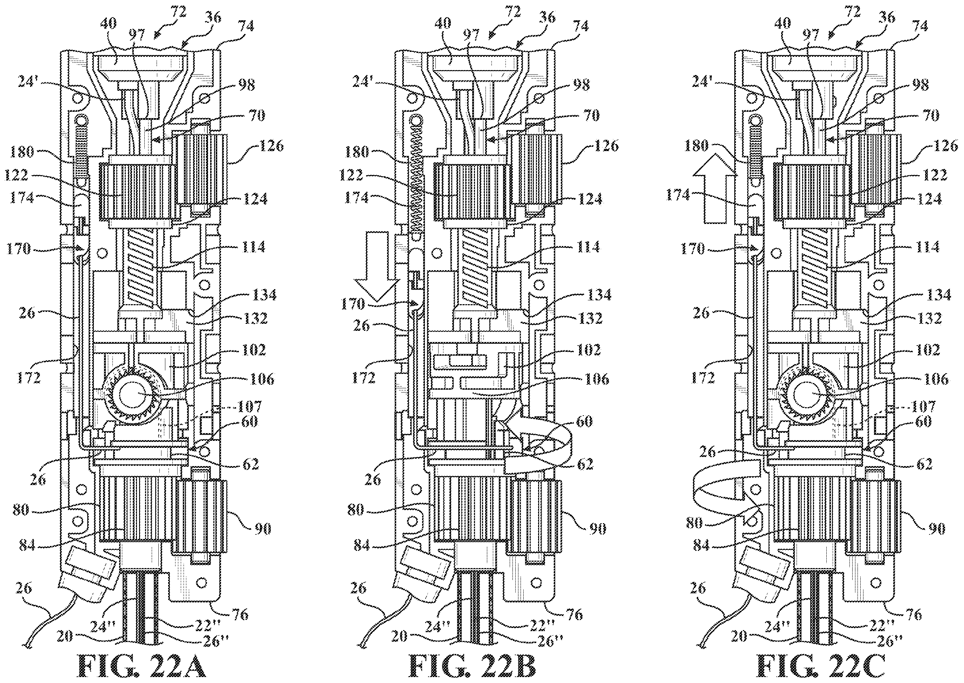

Similar to the winding and overlapping problem discussed immediately above with respect to the dynamic portions of the working and fluid channels **22", 24",** torsion and kinking of the at least one electronics cable **26** during rotation of the catheter shaft **20** can also lead to failure and compromised performance of this working component. Accordingly, the control assembly **10** also includes a cable take-up assembly **60** configured to enable rotation of the catheter shaft **20** without adversely winding the electronics cable **26** and without the additional requirement of a joint and connectors along the electronics cable **26** to achieve this objective, namely because different from the working and fluid channels **22, 24,** it is often not practical to interrupt or bifurcate the wires within the electronics cable **26.** As best illustrated in Figures 2 and 19, the cable take-up assembly **60** is disposed in the control compartment **78** of the control case **72** and is rotatable about the axis **A** synchronously with the union reservoir hub **38** and the center gear **80.** The cable take-up assembly **60** defines a spool **62** extending circumferentially about the axis **A,** and the electronics cable **26** is routed to and around the spool **62** between the static portion **26'** and the dynamic portion **26".** As illustrated in Figures 11A-B and Figures 22B-C, and described in more detail below, synchronous rotation of the cable take-up assembly **60** with the union reservoir hub **38** and the center gear **80** in a first rotational direction winds the electronics cable **36** around the spool **62** and synchronous rotation of the cable take-up assembly **60** in a second opposite rotational direction unwinds the electronics cable **26** from the spool **62** for allowing the dynamic portion **26"** of the electronics cable **26** which passes from the cable take-up assembly **60** to the distal tip **21** of the catheter shaft **20** to maintain a position relative to the dynamic portions of the working and fluid channel components **22", 24"** during their collective, simultaneous rotation with the catheter shaft **20.** As illustrated in Figures 2-4and 9-11B, in accordance with a first embodiment, the cable take-up assembly **60** can be implemented as an integrated component of the lumen interruption mechanism **36,** and thus a combined functionality component. However, as best illustrated in Figures 19-20 and 22A-C, in accordance with a second embodiment, the cable take-up assembly **60** could also be a separate component located elsewhere within the control assembly **10** from the lumen interruption mechanism **36,** in this case on the center gear **80.** This is because in practice the functionality of the cable-take-up assembly **60** is independent of the lumen interruption mechanism **36** (other than the rotational synchronicity) and can exist as a separate component(s) in similar applications.

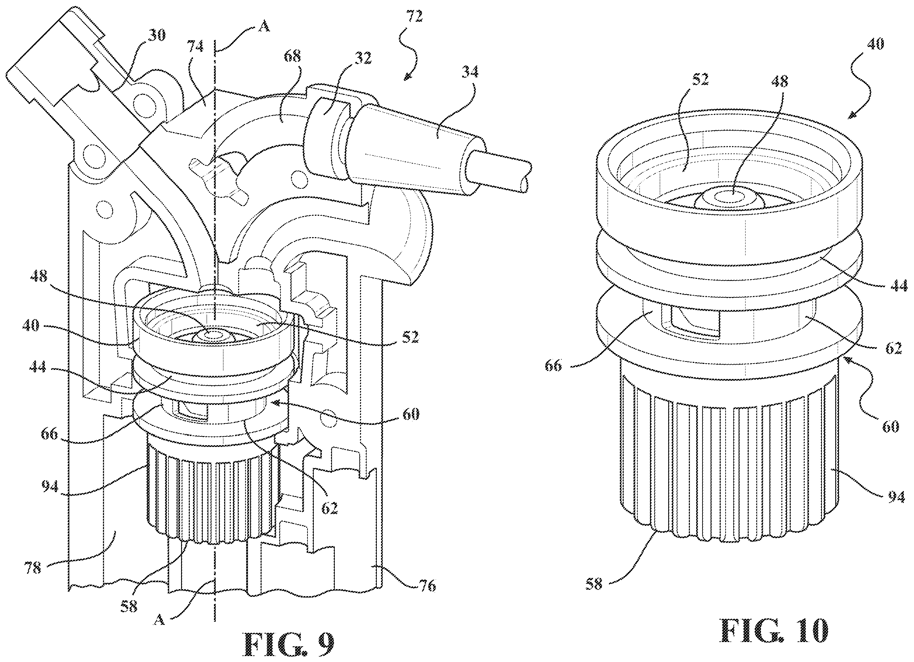

As best illustrated in Figures 9-11, in the first embodiment of the cable take-up assembly **60,** the spool **62** is defined by the union reservoir hub **40** and extends radially inwardly from an exterior surface of the union reservoir hub **40** and circumferentially about the axis **A.** A spool slack chamber **64** is defined between an interior surface of the spool **62** (as defined by an inner diameter of the union reservoir hub **40)** and an interior surface of the control assembly **10,** disposed adjacent the cable take-up assembly **60.** As will be understood in view of the following discussion of operation, the interior surface of the control assembly **10** used to define an exterior wall of the slack chamber **64** provides a barrier to limit the build-up of excessive slack in the slack chamber **64** during rotation of the spool **62.** The spool **62** defines a window **64** disposed in both communication with this slack chamber **66** as well as an electronics cable passage **37** that extends from the lumen interruption mechanism **36** to the electronics port **32.** The electronics cable **26** is routed such that a distal length extends from the distal tip **21** of the catheter shaft **20** to the union reservoir hub **40** of the lumen interruption mechanism **36,** with a medial length of the electronics cable **26** then wound loosely around the spool **62** a predetermined number of times such that the wound electronics cable **26** is housed within the spool slack chamber **66.** A proximal length of the electronics cable **26** then continues from the slack chamber **66,** through the window **64** and extends through the electronics cable passage **37** to be routed through the strain relief **34** and ultimately be affixed to a static PCB. As will be explained above, use of the cable take-up assembly **60** provides for the distal length of the electronics cable **26** to rotate in unison with the catheter shaft **20** such that the electronics cable **26** never winds around other working components, such as the dynamic working and fluid channels **22, 24.** In addition, the cable take-up assembly **60** advantageously allows for the use of one, continuous electronics cable **26** extending through the control assembly 10, without the need for electrical connectors.

As best illustrated in Figure 11, in operation, rotation of the cable take-up assembly **60** in one direction (in this case synchronously with rotation of the union reservoir hub **40)** takes up the slack in the spool **62** to facilitate safe rotation of the catheter shaft **20,** while rotation of the cable take-up assembly **60** in the other direction (again synchronously with corresponding rotation of the union reservoir hub **40)** unwinds and loosens the loops of the electronics cable **26** around the spool **62.** As the cable take-up assembly **60** rotates, the window **66** acts to guide the electronics cable **26** around the spool **62.** Put another way, the window **66** acts like an eyelet, forcing the electronics cable **26** to rotate in association with the union reservoir hub **40.** The number of cable winds corresponds to the amount of limitation required by the control assembly **10,** which can be greater than 360 degrees if necessary, but not practically infinite. As a result, the cable take-up assembly **60** minimizes the torque and winding condition on the electronics cable **26** breaking out from the catheter shaft **20** and which otherwise would occur during rotation of the catheter shaft **20.**

As mentioned previously, in accordance with a second embodiment, the cable take-up assembly **60** is implemented on the center gear **80** as opposed to on the lumen interruption mechanism **36.** (*See* Figures 19-20 and 22A-C). In regards to the electronics cable, there are many types of electronics cables that might be used in the control assembly **10.** Some electronics cables are more resistant to bending, some are more prone to kink, some are more supple and less inclined to react predictably to "push forces", etc. In instances where the mechanical characteristics of the electronics cable do not lend themselves as favorable to the unwinding action described above, particular failure modes might occur. This can manifest in the clockwise (first rotational direction) and counterclockwise (second rotational direction) of the cable take-up assembly **60,** with the back and forth of the electronics cable in tension followed by compression (push) resulting in knotting and accumulation of the electronics cable within the spool **62.** The end effect can be a loss of degradation of image signal due to the knotting, to worst case scenario of the knotting decreasing the total free length of the cable and resulting in fracture of the electronics cable and full loss of function of the electronics.

As best illustrated in Figures 19-23, the control assembly **10** implements a solution to this problem, namely routing the static portion **26'** of the electronics cable **26** through a cable tensioning mechanism **170** prior to the spool **62** to continually apply a small amount of tension to the electronics cable **26** regardless of the rotational direction of the spool **62** and always maintain the electronics cable **26** taught against an inner diameter of the spool **62,** in all instances. In other words, the cable tensioning mechanism **170** keeps the electronic cable **26** in tension with the spool **62,** and prevents the "push" phase of rotation of the spool **62** from bringing in the variability that might result in knotting.

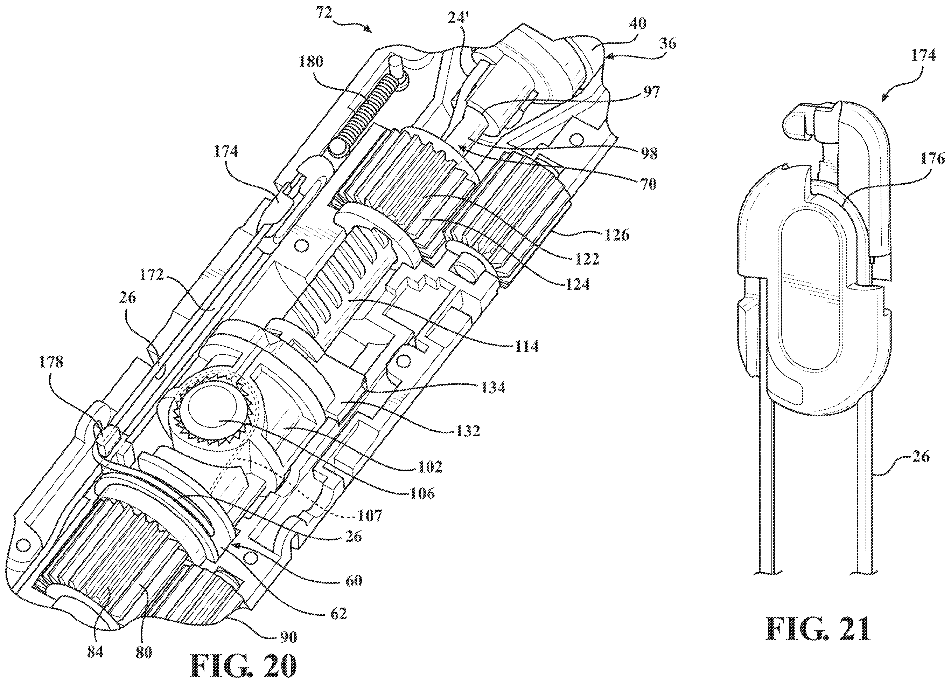

More specifically, as best illustrated in Figures 19-20, the control case **72** defines a tensioning channel **172** extending in generally parallel and radially spaced relationship with the axis **A** between the proximal and distal case ends **74, 76.** When the static portion **26'** of the electronics cable **26** enters the control case **72** via the distal case end **76,** the tensioning channel **172** is disposed in communication with the electronics port **32** and extends from the distal case end **76** towards the proximal case end **74.** However, the directional arrangement of the tensioning channel **172** could be reversed, and extend from the proximal case end **74,** if the electronics cable **26** entered the control case **72** via an electronics port **32** disposed at the proximal case end **74** (such as shown in Figure 2). The cable tensioning mechanism **170** is disposed in the tensioning channel **172** and includes a loop component **174** biased away from the electronics port **32** (in this case towards the proximal case end **74)** and in a direction away from where the static portion **26'** of the electronics cable **26** enters the control case **72.** As best illustrated in Figure 21, the loop component **174** defines a radiused portion **176,** such that the static portion **26'** of the electronics cable **26** is routed from the electronics port **32** through the tensioning channel **172** and to the loop component **174** at which point the electronics cable **26** passes over the radiused portion **176** to redirect the electronics cable from one direction to another and back along the tensioning channel **172** for routing to the spool **62.** As best illustrated in Figure 20, when the electronics cable **26** is routed back adjacent the spool **62,** the electronics cable **26** passes around a shoulder **178** to assist in redirecting the electronics cable **26** transversely from the tensioning channel **172** and towards the spool **62**

As illustrated in Figure 21, the radiused portion **176** of the loop compartment **174** is preferably 180 degrees, to establish that most practical re-direction of force application. However, this redirection of the electronics cable path could be any angle off the major axis to apply a tensile load, but is most effective for direction changes greater than 45 degrees and up to but not including an angle that would put the electronics cable back in line with its major axis, i.e., 360 degrees. The design of this loop component **174** is such that the electronics cable **26** can be installed without the need to pass both ends of the electronics cable **36** through the radiused portion **176,** i.e., the electronics cable **26** can be affixed to the loop component **174** anywhere mid-section of the electronics cable **26.**

As further illustrated in Figures 19-20, the tensioning mechanism **170** includes a biasing member **180,** such as a spring, or the like, extending between the control case **72** and the loop component **174** to establish the biased relationship away from the electronics port **32,** and apply a counter force to the loop component **174** in movement and therefore to the electronics cable **26** routed around the radiused portion **176.** If the biasing member **180** is a tension spring, the tension spring is attached to the loop component **174** above the radiused portion **176** (such as shown in Figures 19-20). However, if the biasing member **180** is a compression spring, the compression spring would be attached below the radiused portion **176,** without departing from the scope of the subject disclosure.

As illustrated in Figures 22A-22C, during operation, rotation of the cable take-up assembly **60** and the associated spool **62** in a first rotational direction (as shown in Figure 22B) winds the electronics cable **26** around the spool **62,** foreshortening a length between the spool **62** and the loop component **174** and causing the loop component **174** to be displaced along the tensioning channel **172** in a direction towards the spool **62.** This displacement is resisted by the biasing member **180** and a tensile force is distributed to the electronics cable **26,** such that the electronics cable **36** is never allowed to have enough slack to entangle or knot. As shown in Figure 22C, changing direction of rotation of the cable take-up assembly **60** and the associated spool **62** in the opposite rotational direction un-wraps the electronic cable **26** from the spool **62** and results in the loop component **174** moving in the opposite linear direction (via the biasing force applied by the biasing member **180),** reducing the tensile force applied to the electronics cable **26.** As the spool **62** passes over center, the direction of the wrap around the spool **62** changes while the loop component **174** repeats its linear cycle applying the tensile load. In this way, as the cable take-up assembly **60** cycles through the full range of rotation, the loop component **174** cycles up and down, like a piston, continually applying a tensile load to the electronics cable **26** to always keep the electronics cable **36** taught against the spool **62** and prevent entanglement and knotting.

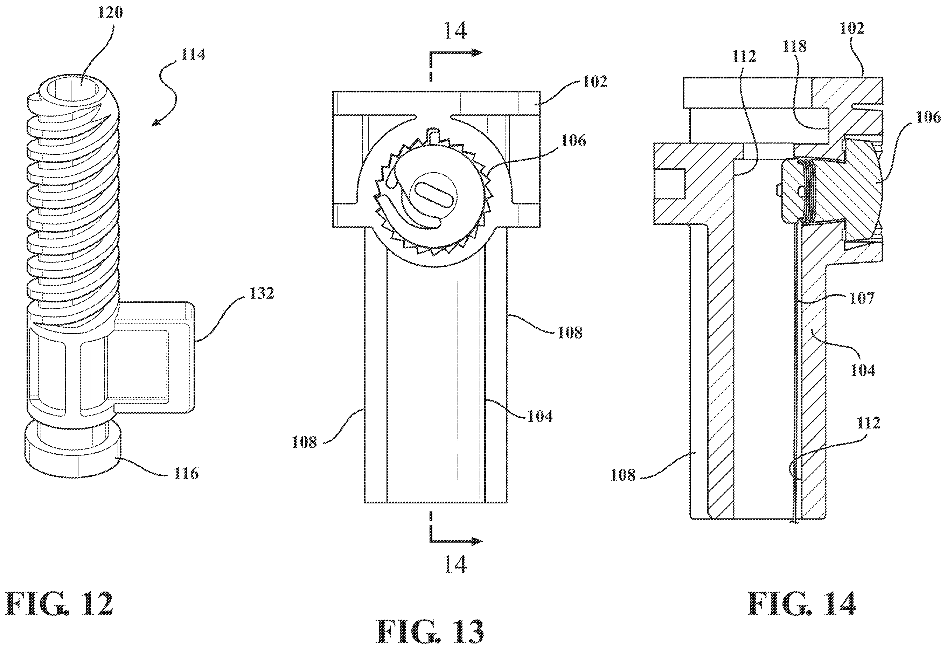

As illustrated in Figures 1 and 1A, the handle **85** of the control assembly **10** also includes a deflection control knob **100** rotatably disposed about the proximal case end **74** of the control case **72** to effectuate deflection of the distal tip **21** of the catheter shaft **20** in response to rotation of the deflection control knob **100** by the user. As best illustrated in Figures 2, 19 and 26B, the control case **72** includes an anchor arm **102** disposed within the control compartment **78** adjacent a proximal end of the center gear **80** and having an anchor shaft **104** extending axially downwardly into the center gear passageway **82.** At least one pull wire **107** extends from the anchor arm **102** into the catheter shaft **20** and ultimately terminates at the distal tip **21.** As best illustrated in Figures 13, 14 and 19, a pull wire tensioning mechanism **106** is provided for fixing the pull wire **107** to the anchor arm **102,** and allowing an operator to modify a length of the pull wire **107** between the anchor arm **102** and the distal tip **21** in order to provide a base tension of the pull wire **107** that correlates with a desired adjustment sensitivity of the pull wire. A preferred arrangement of the pull wire tensioning mechanism **106** is described in U.S. Application Serial No. 17/150,346, such as in Paragraph [0067].

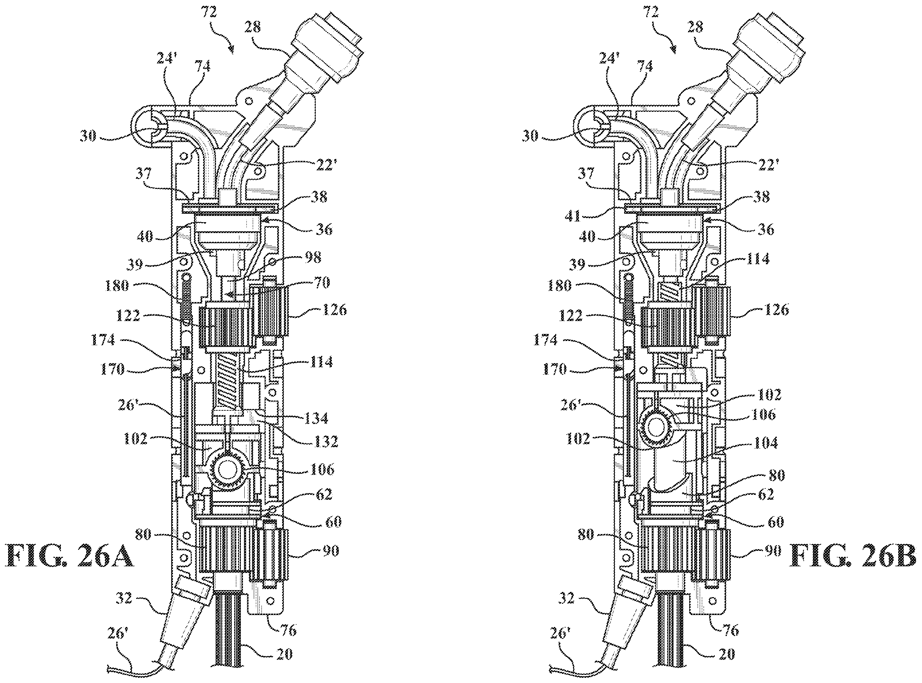

As best illustrated in Figures 2, 13 and 14, the anchor shaft **104** defines at least one axial rib **108** extending radially outwardly from the anchor shaft **102** in aligned and radially spaced relationship with the central axis **A.** The center gear **80** defines at least one axial slot **110** extending radially inwardly from the center gear passageway **82** for receiving the at least one axial rib **108.** Mating of the axial rib **108** with the axial slot **110** drives synchronous and simultaneous rotation of the anchor arm and shaft **102, 104** with the center gear **80,** and also allows the anchor arm and shaft **102, 104** to translate proximally and distally relative to center gear **80** (as will be described in more detail below, and best illustrated in Figures 26A-B) while still maintaining rotational alignment between these components. As a result, the mating of the axial rib **108** with the axial slot **110** allows the anchor arm **102** to work in concert with the center gear **80** as well as the synchronous rotation mechanism **70** in such a way that the pull wire **107** maintains radial orientation with the catheter shaft **20,** and is not buckled, kinked, or overlapped with the other working components during rotation of the catheter shaft **20** by the user via rotation of the rotation control knob **86.**

As illustrated in Figures 13-14, the anchor arm **102** and anchor shaft **104** collectively define an anchor passageway **112** disposed in communication with the center gear passageway **82** for allowing the dynamic portions of the working components of the catheter to be received from the center gear passageway **82** and pass through the anchor passageway **112** into the control compartment **78.** The control case **72** includes a lead screw **114** disposed within the control compartment **78** adj acent to a proximal end of the anchor arm **102** and having a rotor flange **116** disposed in coupled relationship with a circumferential slot **118** defined by an interior portion of the anchor arm **102** and which extends radially inward from the anchor passageway **112.** (See Figure 14). As will be appreciated in view of the following description, this joint holds the lead screw **114** and the anchor arm **102** together axially while also allowing full rotation of the anchor arm relative to the lead screw **114** (which is always maintained in a rotationally static position). As best illustrated in Figure 12, similar to the center gear **80** and the anchor arm **102,** the lead screw **114** also defines a lead screw passageway **120** that allows the dynamic portions of the working components of the catheter to pass along from the anchor passageway **112** towards the union reservoir hub **40** disposed proximally above.

As best illustrated in Figures 1A, 2, 19 and 26A-B, the control case **72** includes a threaded gear **122** disposed in rotationally aligned relationship about the central axis **A** and including an internal thread **123** disposed in threaded relationship with a proximal end of the lead screw **114.** A set of threaded gear teeth **124** extend radially outwardly from the threaded gear **122,** and at least one deflection spur gear **126, 128** is disposed in the control compartment **78** to establish the operably coupled relationship between the deflection control knob **100** and the threaded gear **122.** For example, as illustrated in Figure 19, in accordance with a first arrangement, only a first deflection spur gear **126** is arranged in radially offset relationship from and in operable relationship with both the threaded gear teeth **124** of the threaded gear **122** and the deflection control knob **100** to establish the operable coupling therewith. However, as best illustrated in Figures 1A and 2, in accordance with a second arrangement, an additional second deflection spur gear **128** can be sequentially arranged in radially offset relationship to the threaded gear **122.** Put another way, as best illustrated in Figure 2, the first deflection spur gear **126** is disposed radially offset from and in operable relationship with the threated gear teeth **124** of the threaded gear **122,** and the second deflection spur gear **128** is disposed radially offset from an in operable relationship with the first deflection spur gear **126,** such that rotation of the second (most radially offset) deflection spur gear **128** ultimately drives rotation of threaded gear **122.** As previously mentioned, the handle **85** of the control assembly **10** also includes a deflection control knob **100** rotatably disposed about the proximal case end **74** of the control case **72.** As best illustrated in Figure 1A, this deflection control knob **100** includes a set of deflection knob gear teeth **130** circumferentially arranged along an inner diameter of the deflection control knob **100** and which are operably coupled with the first deflection spur gear **126** (in the first arrangement) or the second deflection spur gear **128** (in the second arrangement).

As best illustrated sequentially in Figures 26A-B, in operation, rotation of the deflection control knob **100** ultimately drives rotation of the threaded gear **122,** which is disposed in engaging relationship with the thread of the lead screw **114,** resulting in axial displacement of the lead screw **114** both proximally and distally (depending on the rotational direction of the deflection control knob **100).** When the lead screw **114** is axially displaced in the proximal direction (as shown in Figure 26B), the anchor arm **102** is also axially displaced over the same distance by way of the coupling between the rotor flange **116** of the lead screw **114** and the circumferential slot **118** of the anchor arm **102,** such that a resultant tension is applied to the pull wire **105** secured to the anchor arm **102** by way of this pulling motion. Thus, this axial displacement of the lead screw **114** over a distance effectuates the desired distal deflection curve in the distal tip **21** of the catheter shaft **20.** For clarity, and as previously mentioned, the mating of the axial ribs and slot **108, 110** allows for axial movement of the anchor arm **102** relative to the center gear **80** and in conjunction with the lead screw **114** to effectuate the desired deflection.

As best illustrated in Figures 1A, 2, 12, 19 and 26A-B, the lead screw **114** includes an anti-torque wing **132** extending radially outwardly from the lead screw **114** and disposed in engaging relationship with a corresponding wing slot **134** disposed adjacent the lead screw **114** and defined by the control case **72** to prevent the lead screw **114** from rotating in response to movement by the threaded gear **122,** and limit movement of the lead screw **114** to only the up (proximal) and down (distal) directions. As a result, torque is not distributed down to the pull wire to further prevent the pull wire from wrapping around other working components of the catheter. A pitch of the lead screw **114** and a pitch of the internal thread **113** of the threaded gear **122** is such that the friction angle remains intact, allowing the lead screw **114** to stay in place static when the deflection control knob **100** is not manipulated by a user.

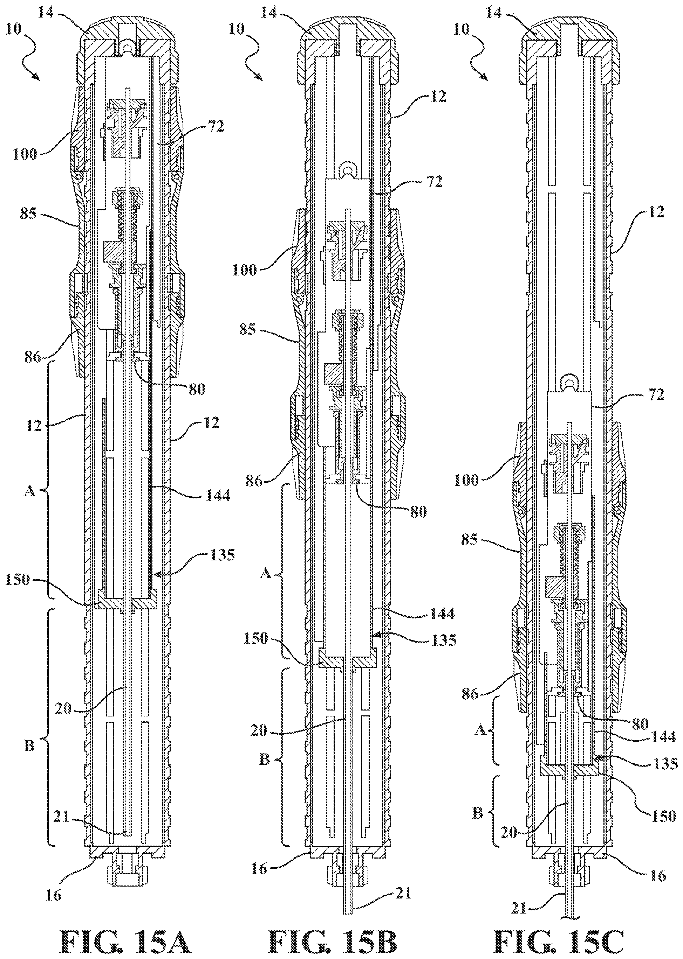

As best illustrated in Figures 15A-C, the control case **72** (which is surrounded by the handle **85)** is axially translatable along the housing **12** from the proximal housing end **14** towards the distal housing end **14** to effectuate advancement and retraction of the catheter shaft **20,** namely because the catheter shaft **20** is affixed to the center gear **80** and travels with the control case **72** during axial movement. As the control case **72** travels towards the distal housing end **14** of the housing **12,** the distal tip **21** of the catheter shaft **20** is axially advanced by the user by way of translating the control case **72.** However, during movement of the control case **72,** a length of the catheter shaft **20** extending between the center gear **80** and the distal end **16** of the housing **12** is inclined to buckle, bend, and/or kink when a compressive load is applied to this length of catheter shaft **20,** such as when the handle **85** is translated along the housing **12** by the user. The catheter shaft **20** has an amount of column strength by design to resist buckling and kinking, however the longer the distance between two points of support, the more side loading due to gravity, angular position of the catheter, user manipulation, etc. is inclined to move the catheter shaft **20** off its central axis A and decrease/defeat column strength.

The control assembly **10** includes a proportional support mechanism **135** disposed within the housing compartment **18** and continuously supporting the catheter shaft **20** at a point between the center gear **80** and the distal housing end **16** during the axial advancement of the catheter shaft **20** to provide additional point(s) of support consistently maintained along the length of catheter shaft **20** extending between the center gear **80** and the distal end **16** of the housing **12** across the entire range of travel of the control case **72** relative to the housing **12.** Put another way, as the control case **72** moves towards the distal end **16** of the housing **12** to axially advance the distal tip **21** of the catheter shaft **20** in the distal direction, the proportional support mechanism **135** moves proportional to this displacement and continuously supports the catheter shaft **20** at a point(s) between the center gear **80** and the distal end **16** of the housing **12** to prevent the catheter shaft **20** from buckling and/or kinking. In a preferred arrangement, the proportional support mechanism **135** continuously supports the catheter shaft **20** at a midpoint between the center gear **80** and the distal housing end **16.** However, other points along the catheter shaft **20** could be utilized without departing from the scope of the subject disclosure.

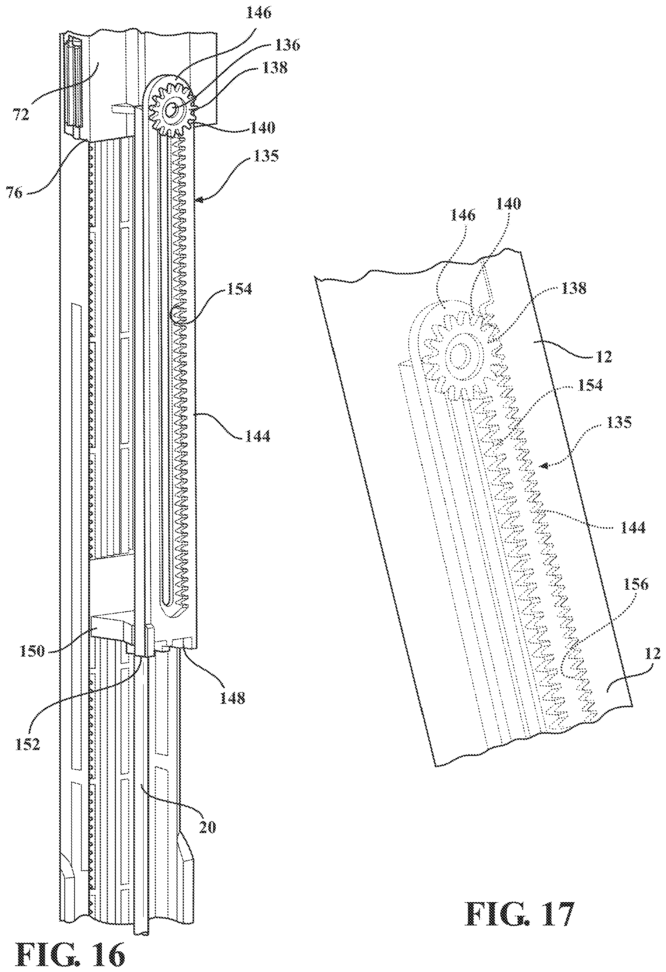

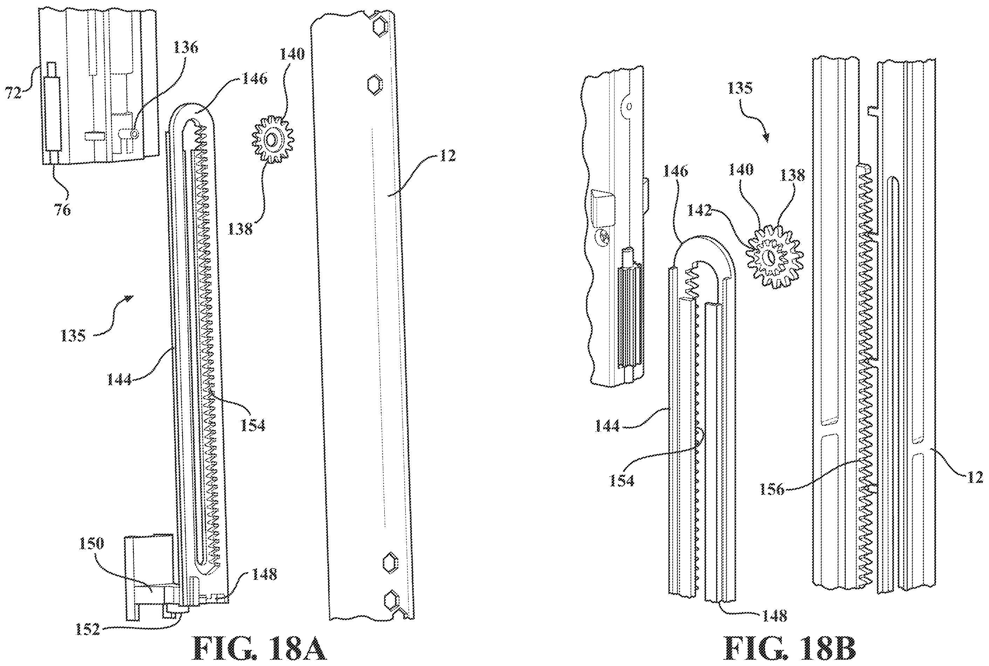

As best illustrated in Figures 16-18, the proportional support mechanism **135** includes a gear boss **136** extending radially outwardly from a distal case end **76** of the control case **72** and a reduction gear **138** having both a major gear feature **140** and a minor gear feature **142** is rotatably disposed on the gear boss **136.** A catheter support arm **144** extends within the compartment **18** of the housing **12** from a first support arm end **146** operably coupled with the minor gear feature **142** of the reduction gear **138** to a second support arm end **148** disposed in spaced relationship with the distal end **16** of the housing **12.** A catheter support platform **150** extends radially from the second support arm end **148** and defines a cathether lumen **152** through which the catheter shaft **20** passes as it extends between the center gear **80** and the distal end **16** of the housing **12.** The catheter support arm **144** defines a minor gear rack **154** extending between the first and second support arm ends **146, 148,** and which is operably coupled with the minor gear feature of the reduction gear **138.** The housing **12** also defines a major gear rack **156** operably coupled with the major gear feature **140** of the reduction gear **138.** The major gear rack **156** extends from a starting position radially adjacent to the reduction gear **138** when the control case **72** is disposed adjacent the proximal housing end **14** to an ending position disposed adjacent the distal housing end **16.**

In operation, since the reduction gear **138** is attached to an axle on the control case **72,** namely the gear boss **136,** linear manipulation of the control case **72** results in rotational engagement of the reduction gear **138** to its mating major and minor gear racks **154, 156.** The ratio of translated linear motion between the major and minor gear racks **154, 156** results in placement of the catheter support platform **150** of the catheter support arm **144** at a central position of the exposed catheter shaft **20.** Put another way, with reference to Figures 15A-C, as the control case **72** is moved towards the distal housing end **16** of the housing **12,** the reductive gear system provided by the reduction gear **138** and the major and minor gear racks **154, 156** maintains a distance between the catheter support platform **150** and the center gear **80** (Distance **A)** and a distance between the catheter support arm **150** and the distal housing end **16** of the housing **12** (Distance **B)** which are always equal. This is because the reduction gear system results in a reduced rate of axial movement of the catheter support arm **144** relative to the control case **72.** In this way, the unsupported lengths of the shaft (Distances **A** and **B)** are always half that of the total unsupported length **(A + B),** promoting column strength by 2X and decreasing the effects of side loading that would otherwise induce buckling and kinking failures. It is understood that this mechanism may be duplicated within an embodiment of the catheter control assembly whereby a plurality of catheter support arms **144,** each with their own reduction gears with specific minor and major gear diameters and associated racks, could be employed to support the catheter shaft at thirds, quarters, etc. in order to best minimize the risk of kinking and buckling over a given catheter length.

Obviously, many modifications and variations of the present invention are possible in light of the above teachings and may be practiced otherwise than as specifically described while within the scope of the appended claims.

## Claims

1. A control assembly (10) for a catheter having at least one working component (22, 24, 26), the control assembly (10) comprising:
a housing (12) extending along an axis (A) between a proximal housing end (14) and a distal housing end (16) to define a housing compartment (18) therebetween;
a control case (72) disposed within said housing compartment (18) adjacent said proximal housing end;
a catheter shaft (20) extending within said housing compartment from said control case to a distal tip (21) and rotatable about the axis during operation of the control assembly;
at least one channel component (22, 24) of the catheter extending from a static portion (22', 24') disposed adjacent said proximal housing end (14) in coupled and stationary relationship relative to said control case (72) to a dynamic portion (22", 24") extending along and rotatable about the axis (A) simultaneously with said catheter shaft (20); and
a lumen interruption mechanism (36) disposed in said control case (72) and extending from a proximal mechanism end (37) disposed in communication with said static portion (22', 24') of said at least one channel component (22, 24) to a distal mechanism end (39) disposed in communication with said dynamic portion (22", 24") of said at least one channel component (22, 24) for transitioning said at least one channel component (22, 24) from said static portion (22', 24') to said dynamic portion (22", 24").

2. The control assembly (10) as set forth in Claim 1, further comprising:
said lumen interruption mechanism (36) includes a cap (18) fixed to said control case (72) and disposed in a static condition relative to said housing (12) and a hub (40) rotatably coupled to said cap and rotatable about the axis (A);
said cap (38) defining at least one cap inlet (46, 50) coupled with said static portion (22', 24') of said at least one channel component (22, 24);
said hub (40) defining at least one hub outlet (48, 56) disposed in communication with said at least one cap inlet (46, 50) and coupled with said dynamic portion (22", 24") of said at least one channel component (22, 24);
wherein said hub (40) rotates simultaneously with said dynamic portion (22", 24") of said at least one channel component (22, 24) during rotation of said catheter shaft (20) to maintain a relative position of said dynamic portion relative to said catheter shaft and prevent kinking of said dynamic portion of said at least one channel component during rotation about the axis (A).

3. The control assembly (10) as set forth in Claim 2, wherein said cap (38) includes a post (41) disposed in mating relationship with said control case (72) to maintain said cap (38) in said static position and a pair of legs (42) extending along an outer portion of said hub (40) and terminating in a rotational channel (44) defined by said hub for establishing said rotatable coupling of said hub (40) and cap (38).

4. The control assembly (10) as set forth in Claim 2, further comprising:
said at least one channel component (22, 24) including a fluid channel component (24) and a working channel component (22) each extending from respective static portions (22', 24') to dynamic portions (22", 24") extending along and rotatable about the axis (A) simultaneously with said catheter shaft (20);
said at least one cap inlet (46, 50) including a central cap inlet (46) extending along the axis (A) and a cap fluid inlet (50) disposed in radially offset relationship with said axis and extending in generally parallel relationship with said central cap inlet;
said central cap inlet (46) coupled to said static portion (22') of said working channel component (22) and said cap fluid inlet (50) coupled to said static portion (24') of said fluid channel component (24);
said at least one hub outlet (48, 56) including a central hub outlet (48) extending along the axis (A) and disposed in fluid communication with said central cap inlet (46) and a hub fluid outlet (56) disposed in radially offset relationship with said axis and in generally parallel relationship with said central hub outlet (48) and in fluid communication with said cap fluid inlet (50); and
said central hub outlet (48) coupled to said dynamic portion (22") of said working channel component (22) and said hub fluid outlet (56) coupled to said dynamic portion (24") of said fluid channel component (24);
wherein said hub (40) rotates simultaneously with both of said dynamic portions (22", 24") of said fluid and working channel components (22, 24) during rotation of said catheter shaft to maintain a position of said dynamic portions relative to one another and prevent winding and overlapping of said dynamic portions of said fluid and working channel components during simultaneous rotation with said catheter shaft (20).

5. The control assembly (10) as set forth in Claim 4, further comprising:
said hub (40) defining a fluid reservoir (52) extending circumferentially about said central hub outlet (48) and disposed adjacent said cap (38) and in continuous fluid communication with said cap fluid inlet (50) during rotation of said hub relative to said cap;
said fluid reservoir (52) disposed in fluid communication with said hub fluid outlet (56) to establish a fluid communication path sequentially via said cap fluid inlet (50), said fluid reservoir (52) and said hub fluid outlet (56); and
at least one sealing device (54) disposed between said hub and said cap to seal said fluid reservoir.

6. The control assembly (10) as set forth in Claim 2, further comprising:
said control case (72) extending from a proximal case end (74) to a distal case end (76) to define a control compartment (78) extending therebetween;
said lumen interruption mechanism (36) disposed within said control compartment (78) adjacent said proximal case end (74);
a center gear (80) disposed in said control compartment (78) adjacent said distal case end (76) and coupled to said catheter shaft (20) for driving simultaneous rotation therewith;
said center gear (80) defining a set of center gear teeth (84) extending radially outwardly from said center gear in circumferentially aligned relationship with the axis (A);
a rotation control knob (86) rotatably disposed about said distal case end (76) and operably coupled with said set of center gear teeth (84) for driving rotation of said center gear (80) and said catheter shaft (20) in response to rotation of said rotation control knob by a user; and
a synchronous rotation mechanism (70) disposed in operably coupled relationship with said hub (40) of said lumen interruption mechanism and said center gear (80) to synchronously drive rotation of said hub in response to rotation of said center gear via said rotation control knob (86).

7. The control assembly (10) as set forth in Claim 6, further comprising:
said synchronous rotation mechanism (70) including an axle (98) extending from a proximal axle end (97) operably coupled with said hub (40) to a distal axle end (99) operably coupled with said center gear (80), said axle rotatable in response to rotation of said center gear to establish said synchronous rotation of said hub.

8. The control assembly (10) as set forth in Claim 7, further comprising:
said axle (98) extending within said control compartment (78) in aligned relationship with said axis (A), and said proximal axle end (97) directly connected to said hub (40) and said distal axle end (99) directly connected to said center gear (80) to establish said synchronous rotation of said hub (40) and said center gear,
wherein said axle (98) defines an internal passageway (101) extending within said axle between said proximal and distal ends (97, 99); and said dynamic portion (22", 24") of said at least one channel component (22, 24) extending from said at least one hub outlet (48, 56), through said internal passageway (101) and into said catheter shaft (20).

9. The control assembly (10) as set forth in Claim 6, further comprising:
a cable take-up assembly (60) disposed in said control compartment (78) of said control case (72) and rotatable about the axis (A) synchronously with said hub (40) and said center gear (80);
said cable take-up assembly (60) defining a spool (62) extending circumferentially about the axis;
an electronics cable (26) of the catheter extending from a static cable portion (26') to a dynamic cable portion (26") extending along and rotatable simultaneously about the axis (A) with said catheter shaft (20); and
said electronics cable routed to said spool of said cable take-up assembly between said static and dynamic cable portions (26', 26");
wherein synchronous rotation of said cable take-up assembly with said hub and said center gear in a first rotational direction winds said electronics cable around said spool and synchronous rotation of said cable take-up assembly with said hub and said center gear in a second opposite rotational direction unwinds said electronics cable from said spool for allowing said dynamic cable portion (26") of said electronics cable (26) to maintain a position relative to said dynamic portion (22", 24") of said at least one channel component (22, 24) during simultaneous rotation with said catheter shaft (20).

10. The control assembly (10) as set forth in Claim 9, wherein said electronics cable (26) is routed through a tensioning mechanism (170) prior to said spool (62) to continuously apply a tension to said electronics cable (26) during rotation of said spool in both the first and second rotational directions and maintain the portion of said electronics cable (26) routed around said spool (62)taught during operation of said cable take-up assembly (60).

11. The control assembly (10) as set forth in Claim 6, further comprising:
said center gear (80) defining a center gear passageway (82) extending along the axis (A);
an anchor arm (102) disposed within said control compartment (78) adjacent said center gear (80) and rotatable about the axis;
said anchor arm (102) having an anchor shaft (104) extending axially into said center gear passageway (82);
at least one pull wire (107) extending from said anchor arm to said distal tip (21) of said catheter shaft (20) for use in deflecting said distal tip;
said center gear (80) defining at least one axial slot (110) extending radially outwardly from said center gear passageway (82); and
said anchor shaft (104) including at least one axial rib (108) extending radially outwardly in aligned and radially spaced relationship with the axis (A) and disposed in mating relationship with said at least one axial slot (110) to drive synchronous rotation of said anchor arm about the axis in response to rotation of said center gear for maintaining a radial orientation of said at least one pull wire (107) relative to said catheter shaft (20).

12. The control assembly (10) as set forth in Claim 11, further comprising:
said anchor arm (102) defining a circumferential slot (118) extending radially inwardly from an anchor passageway (112) extending through said anchor arm (102) and said anchor shaft (104) along the axis (A);
a lead screw (114) disposed within said control compartment (78) adjacent said anchor arm (102);
said lead screw including a rotor flange (116) coupled with said circumferential slot (118) to allow rotation of said anchor arm (102) relative to said lead screw (114) during rotation of said center gear (80);
a threaded gear (122) disposed within the control compartment (78) in threaded relationship with said lead screw (114); and
a deflection control knob (100) rotatably disposed about said proximal case end (74) of said control case (72) and in operably coupled relationship with said threaded gear (122);
wherein rotation of said deflection control knob (100) in a first rotational direction ultimately drives said threaded gear (122) to axially displace said lead screw (114) relative to said threaded gear in a proximal direction, and
wherein said axial displacement of said lead screw in said proximal direction pulls said anchor arm (102) relative to said center gear (80) and slides said at least one axial rib (108) within said at least one axial slot (110) via said coupling of said rotor flange (116) of said anchor arm and said circumferential slot (118) to apply a resultant tension on said at least one pull wire (107) and deflect said distal tip (21) of said catheter shaft (20).

13. The control assembly (10) as set forth in Claim 12, wherein said control case (72) defines a wing slot (134) disposed adjacent said lead screw (114), and wherein said lead screw includes an anti-torque wing (132) extending radially outwardly and disposed in mating relationship with said wing slot (134) to prevent said lead screw from rotating about said axis (A) and limit movement of said lead screw to said proximal direction during said axial movement of said lead screw (114) via said threaded gear (122).

14. The control assembly (10) as set forth in Claim 6, further comprising:
said control case (72) axially translatable along said housing (12) from said proximal housing end (14) towards said distal housing end (16) to axially advance said catheter shaft (20) along the axis (A) and exit said distal tip (21) out of said distal housing end (16); and
a proportional support mechanism (135) disposed within the housing compartment (18) and continuously supporting said catheter shaft (20) at a point between said center gear (80) and said distal housing end (16) during said axial advancement of said catheter shaft (20) to prevent said catheter shaft from buckling or kinking.

15. The control assembly (10) as set forth in Claim 14, further comprising:
said proportional support mechanism (135) including a gear boss (136) extending radially outwardly from said distal case end (76) of said control case (72) and a reduction gear (138) having both a major gear feature (140) and a minor gear feature (142) rotatably disposed on said gear boss (136);
a catheter support arm (144) extending within said housing compartment (18) from a proximal support arm end (146) to a distal support arm end (148) disposed in spaced relationship with said distal housing end (16);
a catheter support platform (150) extending radially from said distal support arm end (148) and defining a catheter lumen (152) supporting said catheter shaft (20) at a midpoint between said center gear (80) and said distal housing end (16);
said catheter support arm (144) defining a minor gear rack (154) operably coupled with said minor gear feature (140) of said reduction gear (138) and extending between said proximal and distal support arm ends (146, 148); and
said housing (12) defining a major gear rack (156) operably coupled with said major gear feature (140) of said reduction gear (138);
wherein said reduction gear (138) and said major and minor gear racks (154, 156) collectively and consistently maintain a distance A extending between said center gear (80) and said catheter support arm (144) being equal to a distance B extending between said catheter support arm (144) and said distal housing end (16) during said axial translation of said control case (72) along said housing (12) to establish continuous support of said catheter shaft (20) by said catheter support arm (144) at a midpoint between said center gear (80) and said distal housing end (16).

## Patentansprüche

1. Steuerungsanordnung (10) für einen Katheter, aufweisend wenigstes ein Arbeitsbauteil (22, 24, 26), wobei die Steuerungsanordnung (10) umfasst:
ein Gehäuse (12), das sich entlang einer Achse (A) zwischen einem proximalen Gehäuseende (14) und einem distalen Gehäuseende (16) erstreckt, um dazwischen einen Gehäuseraum (18) zu definieren;
ein Steuergehäuse (72), das innerhalb des Gehäuseraums (18) benachbart zu dem proximalen Gehäuseende angeordnet ist;
einen Katheterschaft (20), der sich innerhalb des Gehäuseraums von dem Steuergehäuse zu einer distalen Spitze (21) erstreckt und während des Betriebs der Steueranordnung um die Achse drehbar ist;
mindestens ein Kanalbauteil (22, 24) des Katheters, das sich von einem statischen Abschnitt (22', 24'), der benachbart zu dem proximalen Gehäuseende (14) in gekoppelter und stationärer Beziehung relativ zu dem Steuergehäuse (72) angeordnet ist, zu einem dynamischen Abschnitt (22", 24") erstreckt, der sich entlang der Achse (A) erstreckt und gleichzeitig mit dem Katheterschaft (20) um diesen drehbar ist; und
ein Lumen-Unterbrechungsmechanismus (36), der in dem Steuergehäuse (72) angeordnet ist und sich von einem proximalen Mechanismusende (37), das in Verbindung mit dem statischen Abschnitt (22', 24') des mindestens einen Kanalbauteils (22, 24) angeordnet ist, zu einem distalen Mechanismusende (39) erstreckt, das in Verbindung mit dem dynamischen Abschnitt (22", 24") des mindestens einen Kanalbauteils (22, 24) angeordnet ist, um das mindestens eine Kanalbauteil (22, 24) von dem statischen Abschnitt (22', 24') zu dem dynamischen Abschnitt (22", 24") zu überführen.

2. Steuerungsanordnung (10) gemäß Anspruch 1, ferner umfassend:
wobei der Lumen-Unterbrechungsmechanismus (36) eine Kappe (18), die an dem Steuergehäuse (72) befestigt ist und in einem statischen Zustand relativ zu dem Gehäuse (12) angeordnet ist und eine Nabe (40) enthält, die drehbar mit der Kappe gekoppelt und um die Achse (A) drehbar ist;
wobei die Kappe (38) mindestens einen Kappeneinlass (46, 50) definiert, der mit dem statischen Abschnitt (22', 24') des mindestens einen Kanalbauteils (22, 24) gekoppelt ist;
wobei die Nabe (40) mindestens einen Nabenauslass (48, 56) definiert, der in Verbindung mit dem mindestens einen Kappeneinlass (46, 50) angeordnet und mit dem dynamischen Abschnitt (22", 24") des mindestens einen Kanalbauteils (22, 24) gekoppelt ist;
wobei sich die Nabe (40) während der Drehung des Katheterschafts (20) gleichzeitig mit dem dynamischen Abschnitt (22", 24") des mindestens einen Kanalbauteils (22, 24) dreht, um eine relative Position des dynamischen Abschnitts relativ zu dem Katheterschaft aufrechtzuerhalten und ein Knicken des dynamischen Abschnitts mindestens einen Kanalbauteils während der Drehung um die Achse (A) zu verhindern.

3. Steuerungsanordnung (10) gemäß Anspruch 2, wobei die Kappe (38) einen Zapfen (41), der in einer passenden Beziehung zu dem Steuergehäuse (72) angeordnet ist, um die Kappe (38) in der statischen Position zu halten, und ein Paar Beine (42) enthält, die sich entlang eines äußeren Abschnitts der Nabe (40) erstrecken und in einem Drehkanal (44) enden, der durch die Nabe definiert ist, um die drehbare Kupplung der Nabe (40) und der Kappe (38) herzustellen.

4. Steuerungsanordnung (10) gemäß Anspruch 2, ferner umfassend:
wobei das mindestens eine Kanalbauteil (22, 24) ein Fluidkanalbauteil (24) und ein Arbeitskanalbauteil (22) enthält, die sich jeweils von entsprechenden statischen Abschnitten (22', 24') zu dynamischen Abschnitten (22", 24") erstrecken, die sich entlang der Achse (A) erstrecken und gleichzeitig mit dem Katheterschaft (20) um diese drehbar sind;
wobei der mindestens eine Kappeneinlass (46, 50) einen zentraler Kappeneinlass (46), der sich entlang der Achse (A) erstreckt, und einen Kappenfluid-Einlass (50) enthält, die in einer radial versetzten Beziehung zu der Achse angeordnet ist und sich im Allgemeinen parallel zu dem zentralen Kappeneinlass erstreckt;
wobei der zentrale Kappeneinlass (46) mit dem statischen Abschnitt (22') des Arbeitskanal-Bauteils (22) gekoppelt ist und der Kappenfluid-Einlass (50) mit dem statischen Abschnitt (24') des Fluidkanal-Bauteils (24) gekoppelt ist;
wobei der mindestens eine Nabenauslass (48, 56) einen zentralen Nabenauslass (48), der sich entlang der Achse (A) erstreckt und in Fluidverbindung mit dem zentralen Kappeneinlass (46) steht, und einen Nabenfluidauslass (56) umfasst, der in radial versetzter Beziehung zu der Achse und im Wesentlichen parallel zu dem zentralen Nabenauslass (48) angeordnet ist und in Fluidverbindung mit dem Kappenfluidauslass (50) steht; und
wobei der zentrale Nabenauslass (48) mit dem dynamischen Abschnitt (22") dem Arbeitskanal-Bauteil (22) gekoppelt ist und der Nabenfluidauslass (56) mit dem dynamischen Abschnitt (24") des Fluidkanal-Bauteils (24) gekoppelt ist;
wobei sich die Nabe (40) während der Drehung des Katheterschafts gleichzeitig mit beiden dynamischen Abschnitten (22", 24") der Fluid- und Arbeitskanal-Bauteile (22, 24) dreht, um eine Position der dynamischen Abschnitte relativ zueinander aufrechtzuerhalten und ein Wickeln und Überlappen der dynamischen Abschnitte der Fluid- und Arbeitskanal-Bauteile während der gleichzeitigen Drehung mit dem Katheterschaft (20) zu verhindern.

5. Steuerungsanordnung (10) gemäß Anspruch 4, ferner umfassend:
die Nabe (40), die einen Fluidbehälter (52) definiert, der sich in Umfangsrichtung um den zentralen Nabenauslass (48) erstreckt und benachbart zu der Kappe (38) angeordnet ist und während der Drehung der Nabe relativ zu der Kappe in kontinuierlicher Fluidverbindung mit dem Kappenfluideinlass (50) steht;
wobei der Fluidbehälter (52) in Fluidverbindung mit dem Nabenfluidauslass (56) angeordnet ist, um einen Fluidverbindungspfad sequenziell über den Kappenfluideinlass (50), den Fluidbehälter (52) und den Nabenfluidauslass (56) herzustellen; und
mindestens eine Dichtungsvorrichtung (54), die zwischen der Nabe und der Kappe angeordnet ist, um den Fluidbehälter abzudichten.

6. Steuerungsanordnung (10) gemäß Anspruch 2, ferner umfassend:
das Steuergehäuse (72), das sich von einem proximalen Gehäuseende (74) zu einem distalen Gehäuseende (76) erstreckt, um einen Steuerraum (78) zu definieren, der sich dazwischen erstreckt;
den Lumen-Unterbrechungsmechanismus (36), der innerhalb des Steuerraums (78) benachbart zu dem proximalen Gehäuseende (74) angeordnet ist;
ein Mittelzahnrad (80), das in dem Steuerraum (78) benachbart zu dem distalen Gehäuseende (76) angeordnet und mit dem Katheterschaft (20) gekoppelt ist, um eine gleichzeitige Drehung mit diesem zu bewirken;
wobei das Mittelzahnrad (80) einen Satz von Mittelzahnradzähnen (84) definiert, die sich radial nach außen von dem Mittelzahnrad in einer mit der Achse (A) in Umfangsrichtung ausgerichteten Beziehung erstrecken;
ein Drehsteuerknopf (86), der drehbar um das distale Gehäuseende (76) angeordnet und funktionsfähig mit dem Satz von Zahnradzähnen (84) des Mittelzahnrads gekoppelt ist, um die Drehung des Mittelzahnrads (80) und des Katheterschafts (20) in Reaktion auf die Drehung des Drehsteuerknopfs durch einen Benutzer anzutreiben; und
einen Synchronrotationsmechanismus (70), der in funktionsfähiger Verbindung mit der Nabe (40) des Lumen-Unterbrechungsmechanismus und dem Mittelzahnrad (80) angeordnet ist, um die Drehung der Nabe in Reaktion auf Drehung des Mittelzahnrads über den Drehsteuerknopf (86) synchron anzutreiben.

7. Steuerungsanordnung (10) gemäß Anspruch 6, ferner umfassend:
wobei der Synchronrotationsmechanismus (70) eine Achse (98) enthält, die sich von einem proximalen Achsenende (97), das funktionsfähig mit der Nabe (40) gekoppelt ist, zu einem distalen Achsenende (99) erstreckt, das funktionsfähig mit dem Mittelzahnrad (80) gekoppelt ist, wobei die Achse in Reaktion auf eine Drehung des Mittelzahnrads drehbar ist, um die synchrone Drehung der Nabe herzustellen.

8. Steuerungsanordnung (10) gemäß Anspruch 7, ferner umfassend:
die Achse (98) sich innerhalb des Steuerraums (78) in Ausrichtung mit der Achse (A) erstreckt und das proximale Achsenende (97) direkt mit der Nabe (40) verbunden ist und das distale Achsenende (99) direkt mit dem Mittelzahnrad (80) verbunden ist, um die synchrone Drehung der Nabe (40) und des Mittelzahnrads herzustellen,
wobei die Achse (98) einen inneren Durchgang (101) definiert, der sich innerhalb der Achse zwischen dem proximalen und dem distalen Ende (97, 99) erstreckt; und der dynamische Abschnitt (22", 24") des mindestens einen Kanalbauteils (22, 24) erstreckt, die sich von dem mindestens einen Nabenauslass (48, 56) durch den inneren Durchgang (101) und in den Katheterschaft (20) erstreckt.

9. Steuerungsanordnung (10) gemäß Anspruch 6, ferner umfassend:
eine Kabelaufwickelanordnung (60), die in dem Steuerraum (78) des Steuergehäuses (72) angeordnet und synchron mit der Nabe (40) und dem Mittelzahnrad (80) um die Achse (A) drehbar ist;
wobei die Kabelaufwickelanordnung (60) eine Spule (62) definiert, die sich in Umfangsrichtung um die Achse erstreckt;
ein Elektronikkabel (26) des Katheters, das sich von einem statischen Kabelabschnitt (26') zu einem dynamischen Kabelabschnitt (26") erstreckt, der sich entlang des Katheterschafts (20) erstreckt und gleichzeitig um die Achse (A) drehbar ist; und
wobei das Elektronikkabel zwischen dem statischen und dem dynamischen Kabelabschnitt (26', 26") zu der Spule der Kabelaufwickelanordnung geführt ist;
wobei eine synchrone Drehung der Kabelaufwickelanordnung mit der Nabe und dem Mittelzahnrad in einer ersten Drehrichtung das Elektronikkabel um die Spule wickelt und eine synchrone Drehung der Kabelaufwickelanordnung mit der Nabe und dem Mittelzahnrad in einer zweiten entgegengesetzten Drehrichtung das Elektronikkabel von der Spule abwickelt, damit der dynamische Kabelabschnitt (26") des Elektronikkabels (26) während der gleichzeitigen Drehung mit dem Katheterschaft (20) eine Position relativ zu dem dynamischen Abschnitt (22", 24") des mindestens einen Kanalbauteils (22, 24) während gleichzeitiger Drehung mit dem Katheterschaft (20) beizubehalten.

10. Steuerungsanordnung (10) gemäß Anspruch 9, wobei das Elektronikkabel (26) vor der Spule (62) durch einen Spannmechanismus (170) geführt ist, um während der Drehung der Spule sowohl in der ersten als auch in der zweiten Drehrichtung eine Spannung auf das Elektronikkabel (26) auszuüben und den um die Spule (62) geführten Abschnitt des Elektronikkabels (26) während des Betriebs der der Kabelaufwickelanordnung (60) beizubehalten.

11. Steuerungsanordnung (10) gemäß Anspruch 6, ferner umfassend:
ein Mittelzahnrad (80), das einen Mittelzahnraddurchgang (82) definiert, der sich entlang der Achse (A) erstreckt;
einen Verankerungsarm (102), der innerhalb des Steuerraums (78) benachbart zu dem Mittelzahnrad (80) angeordnet und um die Achse drehbar ist;
wobei der Verankerungsarm (102) eine Verankerungswelle (104) aufweist, die sich axial in den Mittelzahnradurchgang (82) erstreckt;
mindestens einen Zugdraht (107), der sich von dem Verankerungsarm zu der distalen Spitze (21) des Katheterschafts (20) erstreckt, um die distale Spitze abzulenken;
wobei das Mittelzahnrad (80) mindestens einen axialen Schlitz (110) definiert, der sich radial nach außen von dem Mittelzahnraddurchgang (82) erstreckt; und
wobei die Verankerungswelle (104) mindestens eine axiale Rippe (108) umfasst, die sich radial nach außen in Ausrichtung und radial beabstandet zu der Achse (A) erstreckt und in Eingriff mit dem mindestens einen axialen Schlitz (110) angeordnet ist, um eine synchrone Drehung des Verankerungsarms um die Achse in Reaktion auf eine Drehung des Mittelzahnrads anzutreiben, um eine radiale Ausrichtung des mindestens einen Zugdrahts (107) relativ zu dem Katheterschaft (20) aufrechtzuerhalten.

12. Steuerungsanordnung (10)gemäß Anspruch 11, ferner umfassend:
den Verankerungsarm (102), der einen Umfangsschlitz (118) definiert, der sich radial nach innen von einem Verankerungsdurchgang (112) erstreckt, der sich durch den Verankerungsarm (102) und die Verankerungswelle (104) entlang der Achse (A) erstreckt;
eine Leitspindel (114), die innerhalb des Steuerraums (78) benachbart zu dem Verankerungsarm (102) angeordnet ist;
wobei die Leitspindel einen Rotorflansch (116) enthält, der mit dem Umfangsschlitz (118) gekoppelt ist, um eine Drehung des Verankerungsarms (102) relativ zu der Leitspindel (114) während der Drehung des Mittelzahnrads (80) zu ermöglichen;
ein Gewindezahnrad (122), das innerhalb des Steuerraums (78) in Gewindeverbindung mit der Leitspindel (114) angeordnet ist; und
ein Ablenkungssteuerknopf (100), der drehbar um das proximale Gehäuseende (74) des Steuergehäuses (72) angeordnet und in Wirkverbindung mit dem Gewindezahnrad (122) steht;
wobei Drehung des Ablenkungssteuerknopfs (100) in einer ersten Drehrichtung letztendlich das Gewindezahnrad (122) antreibt, um die Leitspindel (114) relativ zu dem Gewindezahnrad in einer proximalen Richtung axial zu verschieben, und
wobei die axiale Verschiebung der Leitspindel in der proximalen Richtung den Verankerungsarm (102) relativ zu dem Mittelzahnrad (80) zieht und die mindestens eine axiale Rippe (108) innerhalb des mindestens einen axialen Schlitzes (110) über die Kupplung des Rotorflansches (116) des Verankerungsarms und des Umfangsschlitzes (118) verschiebt, um eine resultierende Spannung auf den mindestens einen Zugdraht (107) auszuüben und die distale Spitze (21) des Katheterschafts (20) auszulenken.

13. Steuerungsanordnung (10) gemäß Anspruch 12, wobei das Steuergehäuse (72) einen Flügelschlitz (134) definiert, der benachbart zu der Leitspindel (114) angeordnet ist, und wobei die Leitspindel einen Anti-Drehmomentflügel (132) enthält, der sich radial nach außen erstreckt und in Eingriff mit dem Flügelschlitz (134) angeordnet ist, um zu verhindern, dass sich die Leitspindel um die Achse (A) dreht, und um die Bewegung der Leitspindel in proximaler Richtung während der axialen Bewegung der Leitspindel (114) über das Gewindezahnrad (122 ) zu begrenzen.

14. Steuerungsanordnung (10) gemäß Anspruch 6, ferner umfassend:
das Steuergehäuse (72), das entlang des Gehäuses (12) von dem proximalen Gehäuseende (14) zu dem distalen Gehäuseende (16) axial verschiebbar ist, um den Katheterschaft (20) entlang der Achse (A) axial vorwärts zu bewegen und die distale Spitze (21) aus dem distalen Gehäuseende (16) herauszuführen; und
einen proportionalen Halterungsmechanismus (135), der innerhalb des Gehäuseraums (18) angeordnet ist und den Katheterschaft (20) während des axialen Vorschubs des Katheterschafts (20) kontinuierlich an einem Punkt zwischen dem Mittelzahnrad (80) und dem distalen Gehäuseende (16) stützt, um ein Knicken oder Verbiegen des Katheterschafts zu verhindern.

15. Steuerungsanordnung (10) gemäß Anspruch 14, ferner umfassend:
wobei der proportionale Stützmechanismus (135) einen Zahnradvorsprung (136), der sich radial nach außen von dem distalen Gehäuseende (76) des Steuergehäuses (72) erstreckt, und ein Untersetzungsgetriebe (138) mit einem großen Zahnradelement (140) und einem kleinen Zahnradelement(142) umfasst, das drehbar auf dem Zahnradvorsprung (136) angeordnet ist;
einen Katheterhalterungsarm (144), der sich innerhalb des Gehäuseraums (18) von einem proximalen Halterungsarmende (146) zu einem distalen Halterungsarmende (148) erstreckt, das in beabstandeter Beziehung zu dem distalen Gehäuseende (16) angeordnet ist;
eine Katheterhalterungsplattform (150), die sich radial von dem distalen Halterungsarmende (148) erstreckt und ein Katheterlumen (152) definiert, das den Katheterschaft (20) an einem Mittelpunkt zwischen dem Mittelzahnrad (80) und dem distalen Gehäuseende (16) hält;
wobei der Katheterhalterungsarm (144) eine kleine Zahnstange (154) definiert, die funktionsfähig mit dem kleinen Zahnradelement (140) des Untersetzungsgetriebes (138) gekoppelt ist und sich zwischen dem proximalen und dem distalen Stützarmende (146, 148) erstreckt; und
wobei das Gehäuse (12) eine große Zahnstange (156) definiert, die funktionsfähig mit dem großen Zahnradelement (140) des Untersetzungsgetriebes (138) gekoppelt ist;
wobei das Untersetzungsgetriebe (138) und die Haupt- und Nebenverzahnungsstangen (154, 156) gemeinsam und konsistent einen Abstand A zwischen dem Mittelzahnrad (80) und dem Katheterhalterungsarm (144) aufrechterhalten, der gleich einem Abstand B zwischen dem Katheterhalterungsarm (144) und dem distalen Gehäuseende (16) während der axialen Verschiebung des Steuergehäuses (72) entlang des Gehäuses (12) ist, um eine kontinuierliche Abstützung des Katheterschafts (20) durch den Katheterhalterarm (144) in einem Mittelpunkt zwischen dem Mittelzahnrad (80) und dem distalen Gehäuseende (16) herzustellen.

## Revendications

1. Ensemble de commande (10) de cathéter ayant au moins une composante de travail (22, 24, 26), l'ensemble de commande (10) comprenant :
un logement (12) s'étendant le long d'un axe (A) entre une extrémité de logement proximale (14) et une extrémité de logement distale (16) pour définir un compartiment de logement (18) entre eux ;
un boîtier de commande (72) disposé à l'intérieur dudit compartiment de logement (18) adjacent à ladite extrémité de logement proximale ;
une tige de cathéter (20) s'étendant à l'intérieur dudit compartiment de logement à partir dudit boîtier de commande vers une pointe distale (21) et rotative sur l'axe pendant le fonctionnement de l'ensemble de commande ;
au moins une composante de canal (22, 24) du cathéter s'étendant d'une partie statique (22', 24') disposée adjacente à ladite extrémité de logement proximale (14) en relation couplée et fixe par rapport audit boîtier de commande (72) vers une partie dynamique (22'', 24") s'étendant le long de et rotative sur l'axe (A) simultanément avec ladite tige de cathéter (20) ; et
un mécanisme d'interruption de lumière (36) disposé dans ledit boîtier de commande (72) et s'étendant d'une extrémité de mécanisme proximale (37) disposée en communication avec ladite partie statique (22', 24') de ladite au moins une composante de canal (22, 24) vers une extrémité de mécanisme distale (39) disposée en communication avec ladite partie dynamique (22", 24'') de ladite au moins une composante de canal (22, 24) pour transitionner ladite au moins une composante de canal (22, 24) de ladite partie statique (22', 24') vers ladite partie dynamique (22'', 24").

2. Ensemble de commande (10) selon la revendication 1, comprenant en outre :
ledit mécanisme d'interruption de lumière (36) incluant un bouchon (18) fixé audit boîtier de commande (72) et disposé dans un état statique par rapport audit logement (12) et un moyeu (40) couplé en rotation audit bouchon et rotatif sur l'axe (A) ;
ledit bouchon (38) définissant au moins une entrée de bouchon (46, 50) couplée à ladite partie statique (22', 24') de ladite au moins une composante de canal (22, 24) ;
ledit moyeu (40) définissant au moins une sortie de moyeu (48, 56) disposée en communication avec ladite au moins une entrée de bouchon (46, 50) et couplée à ladite partie dynamique (22'', 24'') de ladite au moins une composante de canal (22, 24) ;
dans lequel ledit moyeu (40) tourne simultanément avec ladite partie dynamique (22", 24") de ladite au moins une composante de canal (22, 24) pendant la rotation dudit arbre de cathéter (20) pour maintenir une position relative de ladite partie dynamique par rapport à ladite tige de cathéter et empêcher la partie dynamique de vriller de ladite au moins une composante de canal pendant la rotation sur l'axe (A).

3. Ensemble de commande (10) selon la revendication 2, dans lequel ledit bouchon (38) inclut un pilier (41) disposé en relation d'accouplement avec ledit boîtier de commande (72) pour maintenir ledit bouchon (38) dans ladite position statique et une paire de branches (42) s'étendant le long d'une portion extérieure dudit moyeu (40) et finissant dans un canal rotatif (44) défini par ledit moyeu pour établir ledit accouplement rotatif desdits moyeu (40) et bouchon (38).

4. Ensemble de commande (10) selon la revendication 2, comprenant en outre :
ladite au moins une composante de canal (22, 24) incluant une composante de canal fluidique (24) et une composante de canal de travail (22) s'étendant chacun des parties statiques (22', 24') aux parties dynamiques (22", 24") respectives s'étendant le long de et rotatives sur l'axe (A) simultanément avec ladite tige de cathéter (20) ;
ladite au moins une entrée de bouchon (46, 50) incluant une entrée de bouchon centrale (46) s'étendant le long de l'axe (A) et une entrée fluidique de bouchon (50) disposée en relation décalée radialement avec ledit axe et s'étendant dans une relation généralement parallèle avec ladite entrée de bouchon centrale ;
ladite entrée de bouchon centrale (46) couplée à ladite partie statique (22') de ladite composante de canal de travail (22) et ladite entrée fluidique de bouchon (50) couplée à ladite partie statique (24') de ladite composante de canal fluidique (24) ;
ladite au moins une sortie de moyeu (48, 56) incluant une sortie de moyeu centrale (48) s'étendant le long de l'axe (A) et disposée en communication fluidique avec ladite entrée de bouchon centrale (46) et une sortie fluidique de moyeu (56) disposée en relation décalée radialement avec ledit axe et en relation généralement parallèle avec ladite sortie de moyeu centrale (48) et en communication fluidique avec ladite entrée fluidique de bouchon (50) ; et
ladite sortie de moyeu centrale (48) couplée à ladite partie dynamique (22'') de ladite composante de canal de travail (22) et ladite sortie fluidique de moyeu (56) couplée à ladite partie dynamique (24") dudit compartiment de canal fluidique (24) ;
dans lequel ledit moyeu (40) tourne simultanément avec les deux desdites parties dynamiques (22'', 24'') desdites composantes de canal fluidique et de canal de travail (22, 24) pendant la rotation de ladite tige de cathéter pour maintenir une position desdites parties dynamiques l'une par rapport à l'autre et empêcher l'enroulement et le chevauchement desdites parties dynamiques desdites composantes de canal fluidique et de canal de travail pendant la rotation simultanée avec ladite tige de cathéter (20).

5. Ensemble de commande (10) selon la revendication 4, comprenant en outre que :
ledit moyeu (40) définit un réservoir fluidique (52) s'étendant de manière circonférentielle autour de ladite sortie de moyeu centrale (48) et disposée adjacente audit bouchon (38) et en communication fluidique continue avec ladite entrée fluidique de bouchon (50) pendant la rotation dudit moyeu par rapport audit bouchon ;
ledit réservoir fluidique (52) disposé en communication fluidique avec ladite sortie fluidique de moyeu (56) pour établir un trajet de communication fluidique de manière séquentielle par le biais de ladite entrée fluidique de bouchon (50), ledit réservoir de fluide (52) et ladite sortie fluidique de moyeu (56) ; et
au moins un dispositif d'étanchéité (54) disposé entre ledit moyeu et ledit bouchon pour fermer ledit réservoir fluidique de manière étanche.

6. Ensemble de commande (10) selon la revendication 2, comprenant en outre :
ledit boîtier de commande (72) s'étendant d'une extrémité de boîtier proximale (74) à une extrémité de boîtier distale (76) pour définir un compartiment de commande (78) s'étendant entre eux ;
ledit mécanisme d'interruption de lumière (36) disposé à l'intérieur dudit compartiment de commande (78) adjacent à ladite extrémité de boîtier proximale (74) ;
un engrenage central (80) disposé dans ledit compartiment de commande (78) adjacent à ladite extrémité de boîtier distale (76) et couplé à ladite tige de cathéter (20) pour entraîner la rotation simultanée avec celle-ci ;
ledit engrenage central (80) définissant un jeu de dents d'engrenage central (84) s'étendant radialement vers l'extérieur à partir dudit engrenage central dans une relation alignée de manière circonférentielle avec l'axe (A) ;
une molette de commande de rotation (86) disposée de manière rotative autour de ladite extrémité de boîtier distale (76) et couplée de manière opérationnelle avec ledit jeu de dents d'engrenage central (84) pour entraîner en rotation ledit engrenage central (80) et ladite tige de cathéter (20) en réponse à la rotation de ladite molette de commande de rotation par un utilisateur ; et
un mécanisme de rotation synchrone (70) disposé en relation couplée de manière opérationnelle avec ledit moyeu (40) dudit mécanisme d'interruption de lumière et ledit engrenage central (80) pour entraîner ledit moyeu en rotation de manière synchrone en réponse à la rotation dudit engrenage central par le biais de ladite molette de commande de rotation (86).

7. Ensemble de commande (10) selon la revendication 6, comprenant en outre :
ledit mécanisme de rotation synchrone (70) incluant un axe (98) s'étendant d'une extrémité d'axe proximale (97) couplée de manière opérationnelle audit moyeu (40) vers une extrémité d'axe distale (99) couplée de manière opérationnelle audit engrenage central (80), ledit axe étant rotatif en réponse à la rotation dudit engrenage central pour établir ladite rotation synchrone dudit moyeu.

8. Ensemble de commande (10) selon la revendication 7, comprenant en outre :
ledit axe (98) s'étendant à l'intérieur dudit compartiment de commande (78) en relation alignée avec ledit axe (A), et ladite extrémité d'axe proximale (97) connectée directement audit moyeu (40) et ladite extrémité d'axe distale (99) connectée directement audit engrenage central (80) pour établir ladite rotation synchrone dudit moyeu (40) et dudit engrenage central,
dans lequel ledit axe (98) définit un passage interne (101) s'étendant à l'intérieur dudit axe entre lesdites extrémités proximale et distale (97, 99) ; et ladite partie dynamique (22" , 24") de ladite au moins une composante de canal (22, 24) s'étendant de ladite au moins une sortie de moyeu (48, 56) à travers ledit passage intérieur (101) et jusque dans ladite tige de cathéter (20).

9. Ensemble de commande (10) selon la revendication 6, comprenant en outre :
un ensemble d'enroulement de câble (60) disposé dans ledit compartiment de commande (78) de boîtier de commande (72) et rotatif sur l'axe (A) de manière synchrone avec ledit moyeu (40) et ledit engrenage central (80) ;
ledit ensemble d'enroulement de câble (60) définissant une bobine (62) s'étendant de manière circonférentielle sur l'axe ;
un câble d'électronique (26) du cathéter s'étendant d'une partie de câble statique (26') à une partie de câble dynamique (26'') s'étendant le long de et pouvant tourner simultanément sur l'axe (A) avec ladite tige de cathéter (20) ; et
ledit câble d'électronique étant routé vers ladite bobine dudit ensemble d'enroulement de câble entre lesdites parties de câble statique et dynamique (26', 26'') ;
dans lequel la rotation synchrone dudit ensemble d'enroulement de câble avec ledit moyeu et ledit engrenage central dans une première direction de rotation enroule ledit câble d'électronique autour de ladite bobine et la rotation synchrone dudit ensemble d'enroulement de câble avec ledit moyeu et ledit engrenage central dans une seconde direction de rotation opposée déroule ledit câble d'électronique de ladite bobine pour permettre à ladite partie de câble dynamique (26'') dudit câble d'électronique (26) de maintenir une position par rapport à ladite partie dynamique (22", 24'') de ladite au moins une composante de canal (22, 24) pendant la rotation simultanée avec ladite tige de cathéter (20).

10. Ensemble de commande (10) selon la revendication 9, dans lequel ledit câble d'électronique (26) est routé à travers un mécanisme de tension (170) avant ladite bobine (62) pour appliquer une tension en continu sur ledit câble d'électronique (26) pendant la rotation de ladite bobine dans les deux première et seconde directions de rotation et maintenir la partie dudit câble d'électronique (26) routée autour de ladite bobine (62) prise pendant le fonctionnement dudit ensemble d'enroulement de câble (60).

11. Ensemble de commande (10) selon la revendication 6, comprenant en outre :
ledit ensemble central (80) définissant un passage d'engrenage central (82) s'étendant le long de l'axe (A) ;
un bras d'ancrage (102) disposé à l'intérieur dudit compartiment de commande (78) adjacent audit engrenage central (80) et pouvant tourner sur l'axe ;
ledit bras d'ancrage (102) ayant une tige d'ancrage (104) s'étendant axialement jusque dans ledit passage d'engrenage central (82) ;
au moins un câble de traction (107) s'étendant dudit bras d'ancrage vers ladite pointe distale (21) de ladite tige de cathéter (20) pour l'utilisation dans la déviation de ladite pointe distale ;
ledit engrenage central (80) définissant au moins une fente axiale (110) s'étendant radialement vers l'extérieur à partir dudit passage d'engrenage central (82) ; et
ladite tige d'ancrage (104) incluant au moins une nervure axiale (108) s'étendant radialement vers l'extérieur dans une relation alignée et radialement espacée avec l'axe (A) et disposée en relation d'accouplement avec ladite au moins une fente axiale (110) pour entraîner la rotation synchrone dudit bras d'ancrage sur l'axe en réponse à la rotation dudit engrenage central pour maintenir une orientation radiale dudit au moins un câble de traction (107) par rapport à ladite tige de cathéter (20).

12. Ensemble de commande (10) selon la revendication 11, comprenant en outre :
ledit bras d'ancrage (102) définissant une fente circonférentielle (118) s'étendant radialement vers l'intérieur à partir d'un passage d'ancrage (112) s'étendant à travers ledit bras d'ancrage (102) et ladite tige d'ancrage (104) le long de l'axe (A) ;
une vis-mère (114) disposée à l'intérieur dudit compartiment de commande (78) adjacente audit bras d'ancrage (102) ;
ladite vis-mère incluant une bride de rotor (116) couplée à ladite fente circonférentielle (118) pour permettre la rotation dudit bras d'ancrage (102) par rapport à ladite vis-mère (114) pendant la rotation dudit engrenage central (80) ;
un engrenage fileté (122) disposé à l'intérieur du compartiment de commande (78) en relation filetée avec ladite vis-mère (114) ; et
une molette de commande de déviation (100) disposée en rotation autour de ladite extrémité de boîtier proximale (74) dudit boîtier de commande (72) et en relation couplée de manière opérationnelle avec ledit engrenage fileté (122) ;
dans lequel la rotation de ladite mollette de commande de décalage (100) dans une première direction de rotation entraîne enfin ledit engrenage fileté (122) pour déplacer axialement ladite vis-mère (114) par rapport audit engrenage fileté dans une direction proximale, et
dans lequel ledit déplacement axial de ladite vis-mère dans ladite direction proximale tire ledit bras d'ancrage (102) par rapport audit engrenage central (80) et glisse ladite au moins une nervure axiale (108) à l'intérieur de ladite au moins une fente axiale (110) par ledit accouplement de ladite bride de rotor (116) dudit bras d'ancrage et ladite fente circonférentielle (118) pour appliquer une tension résultante sur ledit au moins un câble de traction (107) et dévier ladite pointe distale (21) de ladite tige de cathéter (20).

13. Ensemble de commande (10) selon la revendication 12, dans lequel ledit boîtier de commande (72) définit une fente à ailettes (134) disposée adjacente à ladite vis-mère (114), et dans lequel ladite vis-mère inclut une ailette anti-couple de rotation (132) s'étendant radialement vers l'extérieur et disposée en relation d'accouplement avec ladite fente à ailettes (134) pour empêcher ladite vis-mère de tourner sur ledit axe (A) et limiter le mouvement de ladite vis-mère vers ladite direction proximale pendant ledit mouvement axial de ladite vis-mère (114) par le biais dudit engrenage fileté (122).

14. Ensemble de commande (10) selon la revendication 6, comprenant en outre :
ledit boîtier de commande (72) pouvant translater axialement le long dudit logement (12) de ladite extrémité de logement proximale (14) vers ladite extrémité de logement distale (16) pour faire avancer ladite tige de cathéter (20) axialement le long de l'axe (A) et faire sortir ladite pointe distale (21) hors de ladite extrémité de logement distale (16) ; et
un mécanisme de support proportionnel (135) disposé à l'intérieur du compartiment de logement (18) et supportant en continu ladite tige de cathéter (20) à un point entre ledit engrenage central (80) et ladite extrémité de logement distale (16) pendant ledit avancement axial de ladite tige de cathéter (20) pour empêcher que ladite tige de cathéter ne fasse une boucle ou ne s'entortille.

15. Ensemble de commande (10) selon la revendication 14, comprenant en outre :
ledit mécanisme de support proportionnel (135) incluant un bossage d'engrenage (136) s'étendant radialement vers l'extérieur à partir de ladite extrémité de boîtier distale (76) dudit boîtier de commande (72) et un engrenage réducteur (138) ayant à la fois une caractéristique d'engrenage principal (140) et une caractéristique d'engrenage secondaire (142) disposées de manière à pouvoir tour sur ledit bossage d'engrenage (136) ;
un bras de support de cathéter (144) s'étendant à l'intérieur dudit compartiment de logement (18) d'une extrémité de bras de support proximale (146) à une extrémité de bras de support distale (148) disposé en relation espacée avec ladite extrémité de logement distale (16) ;
une plateforme de support de cathéter (150) s'étendant radialement de ladite extrémité de bras de support distale (148) et définissant une lumière de cathéter (152) supportant ladite tige de cathéter (20) à un point médian entre ledit engrenage central (80) et ladite extrémité de logement distale (16) ;
ledit bras de support de cathéter (144) définissant une crémaillère d'engrenage secondaire (154) couplée de manière opérationnelle avec ladite caractéristique d'engrenage secondaire (140) dudit engrenage réducteur (138) et s'étendant entre lesdites extrémités de bras de support proximale et distale (146 ; 148) ; et
ledit logement (12) définissant une crémaillère d'engrenage principal (156) couplée de manière opérationnelle à ladite caractéristique d'engrenage principal (140) dudit engrenage réducteur (138) ;
dans lequel ledit engrenage réducteur (138) et lesdites crémaillères d'engrenage principal et secondaire (154, 156) maintiennent collectivement et constamment une distance A s'étendant entre ledit engrenage central (80) et ledit bras de support de cathéter (144) étant égale à une distance B s'étendant ledit bras de support de cathéter (144) et ladite extrémité de logement distale (16) pendant ladite translation axiale dudit boîtier de commande (72) le long dudit logement (12) pour établir un support continu de ladite tige de cathéter (20) par ledit bras de support de cathéter (144) à un point médian entre ledit engrenage central (80) et ladite extrémité de logement distale (16).
